# EUROPEAN PATENT APPLICATION

(11) **EP 4 781 970 A1**
(43) Date of publication of application: **29.07.2026**
(21) Application number: 25154530.7
(22) Date of filing: 28.01.2025
(51) Int. Cl.: A61K 6/17, A61K 6/77, A61K 6/836, A61K 6/887

(54) **SMART DENTAL COMPOSITES WITH ADAPTABLE CONSISTENCY**

(71) Applicant: Ivoclar Vivadent AG, 9494 Schaan (LI)
(72) Inventor: Catel, Yohann, 9475 Sevelen (CH); Grob, Benjamin, 9470 Buchs (CH)
(74) Representative: Uexküll & Stolberg

(57) **Abstract**

The invention relates to a radically polymerizable dental composition comprising (a) at least one urethane di(meth)acrylate monomer; (b) at least one aromatic di(meth)acrylate monomer; (c) at least one inorganic filler; and (d) at least one photoinitiator system for the radical polymerization. The inorganic filler (c) has an average particle size within the range of from 0.15 to 1.5 µm and is surface-modified with a silane according to formula (I):

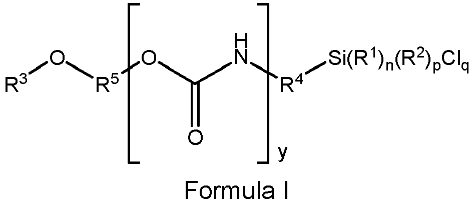

## Description

### TECHNICAL FIELD

The present invention relates to radically polymerizable compositions, which are particularly suitable as dental materials for restoring teeth and which combine the properties of a flowable and a packable composite.

### BACKGROUND

Dental composites are esthetic materials that are widely used in restorative dentistry. Such materials are made up of a mixture of dimethacrylates, additives (photoinitiator system, stabilizer, etc.) and inorganic fillers. 2,2-Bis[4-(2-hydroxy-3-methacryloyloxy-propoxy)-phenyl]propane (Bis-GMA) and bis-[(2-methacryloyloxyethoxy-carbonyl)-amino]-2,2,4-trimethylhexane (UDMA) are frequently used as crosslinking monomers in dental composites. In order to reduce the viscosity of the monomer mixture, low viscosity diluents, such as triethylene glycol dimethacrylate (TEGDMA) or 1,10-decanediol dimethacrylate are added. Radiopaque barium or strontium glass fillers, mixed oxides, such as SiO₂ and ZrO₂, silica nanoparticles and ytterbium trifluoride are commonly used as inorganic fillers. With the exception of ytterbium trifluoride, these fillers are functionalized with a silane bearing a methacrylate group, usually 3-(trimethoxysilyl)propyl methacrylate.

Depending on their consistency, dental composites are divided into packable and flowable composites. Packable composites are highly viscous materials that contain a high amount of inorganic fillers and are suitable for the modeling of the occlusal surface. Due to the high filler content, these materials exhibit a high flexural modulus and a relatively low volumetric shrinkage. On the other hand, flowable composites contain a lower amount of fillers and have a lower viscosity. These materials are able to flow and are used as cavity liners as they can adapt to the cavity walls. In comparison to packable composites, they exhibit a lower flexural modulus and a higher polymerization shrinkage.

For direct filling therapy, the dentist usually needs both materials. First, a flowable material that can adapt to the cavity walls is placed in the cavity and then a packable composite material is used to fill the cavity. To simplify tooth restoration, materials have been proposed that combine the properties of flowable and packable composites in a single material.

WO 2019/123333 A1 discloses dental compositions that include a flowable part A comprising a reactant A and a flowable part B comprising a reactant B. For use, part A and part B are mixed. The mixed composition maintains a flowable viscosity for a time sufficient for the mixed composition to be applied to a dental surface. When mixed, reactant A and reactant B interact, e.g. by an acid-base reaction, which causes an increase in viscosity so that the material can be sculpted and/or shaped. After sculpting and/or shaping, the composition is cured by free radical polymerization.

WO 2019/243339 A1 discloses light-curable single-component dental composite compositions that have a viscosity of more than 400 Pas at 20°C and less than 150 Pas at 50°C. The exemplary compositions contain at least about 50 wt.% of a glass filler with an average particle size (D₅₀) of 2.7 µm. Fillers with a large particle size allow the addition of large amounts of filler without increasing the viscosity excessively. However, large filler particles are disadvantageous because the cured materials tend to have relatively rough surfaces.

### SUMMARY

It is an object of the present invention to provide dental composites, that can be used as flowable composites and as packable composites and which achieve mechanical properties comparable to those achieved with separate packable and flowable composites, in particular good flexural strength and flexural modulus.

### DETAILED DESCRIPTION

According to the invention, this object is achieved by radically polymerizable compositions which comprise
(a) at least one urethane di(meth)acrylate monomer;
(b) at least one aromatic di(meth)acrylate monomer;
(c) at least one inorganic filler; and
(d) at least one photoinitiator for the radical polymerization.

The materials are characterized in that the inorganic filler (c) has an average particle size within the range of from 0.15 to 1.5 µm and is surface-modified with a silane according to formula (I): 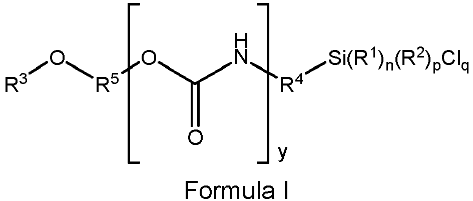 wherein
- R¹: is a OCH₃ or a OC₂H₅ group,
- R²: is a CH₃ or a C₂H₅ group,
- R³: is a group according to formula (II), wherein R⁶ is H or preferably CH₃, or a group according to formula (III), wherein R⁷ is a C₁-C₈ alkyl group, preferably a C₁-C₆ alkyl group, and more preferably a C₁-C₄ alkyl group,

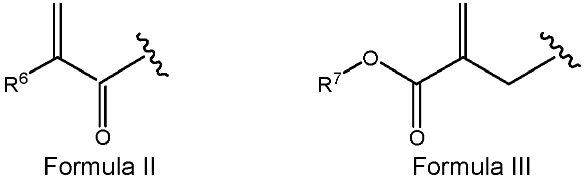

- R⁴: is a linear or branched C₂-C₁₀ alkanediyl group, preferably a C₂-C₆ alkanediyl group, and more preferably a C₂-C₄ alkanediyl group,
- R⁵: is a linear or branched C₆-C₁₄ alkanediyl group, preferably a C₆-C₁₂ alkanediyl group, and more preferably a C₆-C₁₀ alkanediyl group,
- n =: 0, 1, 2 or 3,
- p =: 0, 1 or 2,
- q =: 0, 1, 2 or 3,
- y =: 0 or 1, and
- n + p + q =: 3.

It was surprisingly found that materials comprising an inorganic filler with the above particle size that is surface-modified with a silane of formula (I), in combination with monomers (a) and (b), are packable at a temperature of 30°C or less, preferably 15 to 30°C, more preferably 20 to 30°C, and are flowable at a temperature of 60°C or more, preferably 60 to 80°C and more preferably 60 to 70°C. At a temperature above about 60°C, the materials behave like a flowable composite and can be used by a dentist in the usual way, just like an ordinary flowable material. On cooling, the consistency of the materials changes and they can be shaped and sculpted like a packable composite. This means that only a single material is required for the treatment of a tooth. A measure of the consistency and flowability of the materials is the compressive force. The materials have a compressive force, measured with a texture analyzer as described in the examples, of ≥ 500 g, preferably 500 to 5000 g, at 30°C and ≤ 60 g, preferably 7 to 60 g, at 60°C.

### MONOMERS (A) AND (B)

The radically polymerizable material according to the present invention comprise at least one **urethane di(meth)acrylate monomer (a)**, preferably a di(meth)acrylate monomer with one or two urethane groups. The urethane di(meth)acrylate monomer (a) does not contain aromatic groups.

Particularly preferred are urethane di(meth)acrylate monomers according to **formula (IV):** 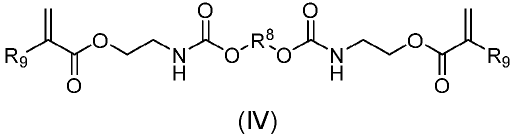 wherein
- R⁸: is a branched or linear C₂-C₁₂ alkanediyl group, preferably a C₂-C₁₀ alkanediyl group, and more preferably a C₂-C₈ alkanediyl group, that can be interrupted by one or more O atoms and/or -C(O)O- groups, and
- R⁹: is H or preferably CH₃;
and/or **formula (V):** 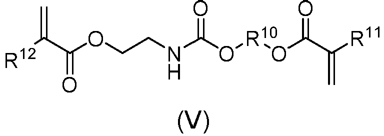 wherein
- R¹⁰: is a branched or linear C₂-C₁₀ alkanediyl group, preferably a C₂-C₈ alkanediyl group, and more preferably a C₂-C₆ alkanediyl group, that can be interrupted by one or more O atoms and/or -C(O)O- groups,
- R¹¹: is H or preferably CH₃, and
- R¹²: is H or preferably CH₃;
and/or **formula (VI):** 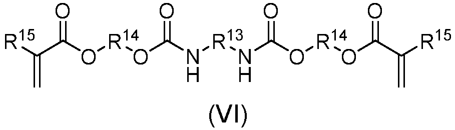 wherein
- R¹³: is a branched or a linear C₂-C₁₆ alkanediyl group or a C₆-C₂₀ cycloaliphatic group, preferably a C₄-C₁₂ alkanediyl group or a C₆-C₁₆ cycloaliphatic group, and more preferably a C₅-C₁₀ alkanediyl group or a C₆-C₁₃ cycloaliphatic group,
- R¹⁴: is a branched or linear C₂-C₁₀ alkanediyl group, preferably a C₂-C₈ alkanediyl group, and more preferably a C₂-C₆ alkanediyl group, that can be interrupted by one or more O atoms and/or -C(O)O- groups, and
- R¹⁵: is H or preferably CH₃.

Urethane di(meth)acrylate monomers according to formulas (IV) and (VI) comprise two urethane groups, and urethane di(meth)acrylate monomers according to formula (V) comprise only one urethane group.

Urethane di(meth)acrylate monomers according to **formula (IV)** can be synthesized via the reaction of a diol with 2-isocyanatoethyl (meth)acrylate.

Particularly preferred monomers according to formula (IV) are:

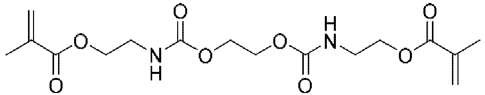

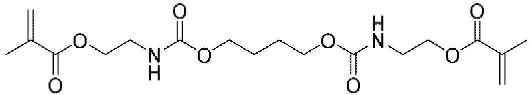

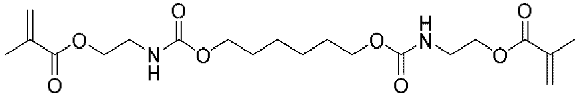

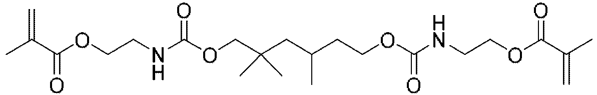

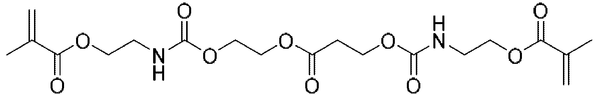

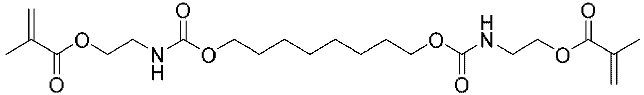

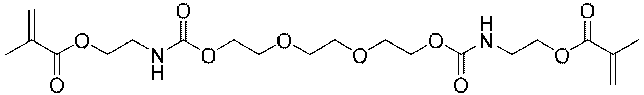

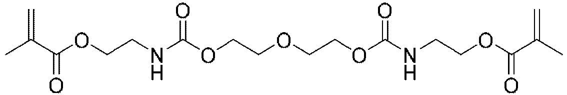

Urethane di(meth)acrylate monomers according to **formula (V)** comprise only one urethane group. They can be synthesized via the reaction of a hydroxyalkyl (meth)acrylate with 2-isocyanatoethyl (meth)acrylate.

Particularly preferred monomers according to formula (V) are:

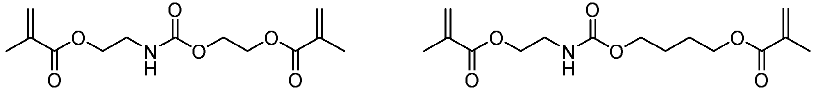

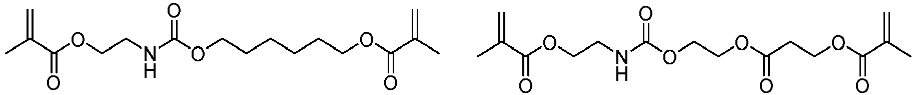

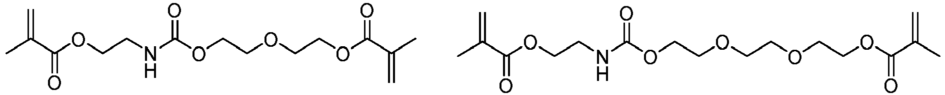

Urethane di(meth)acrylate monomers according to **formula (VI)** comprise two urethane groups. They can be synthesized via the reaction of a hydroxyalkyl (meth)acrylate with a diisocyanate. Preferred diisocyanates for the synthesis of monomer according to formula (VI) are hexamethylene diisocyanate, trimethylhexamethylene diisocyanate, pentamethylene diisocyanate, isophorone diisocyanate and bis (4-isocyanatocyclohexyl)methane.

Particularly preferred monomers according to formula (VI) are:

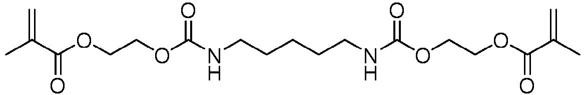

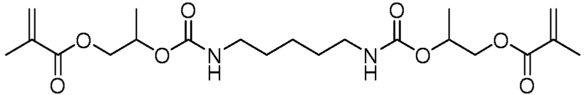

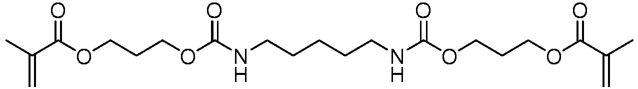

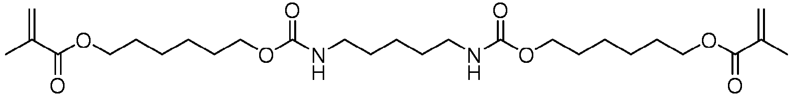

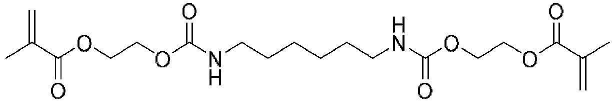

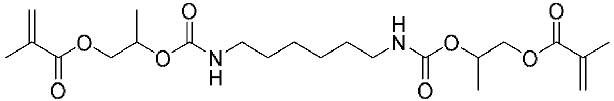

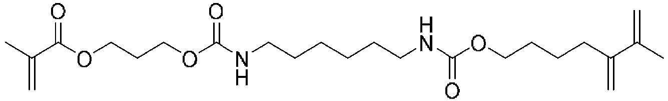

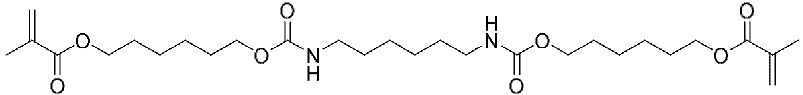

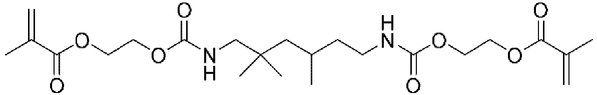

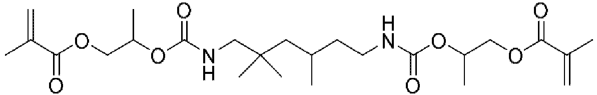

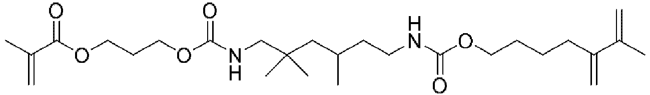

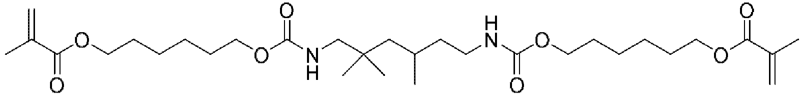

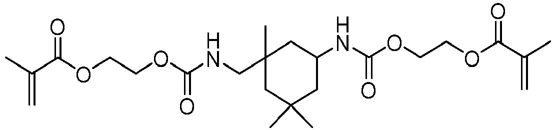

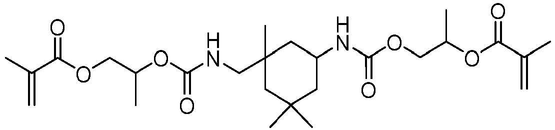

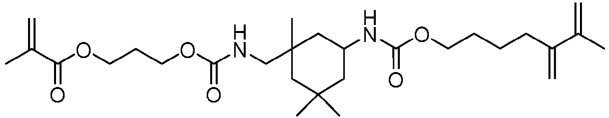

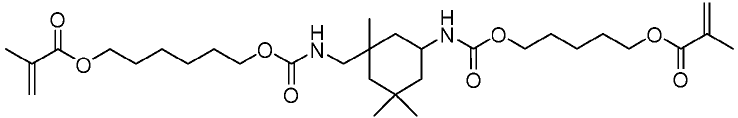

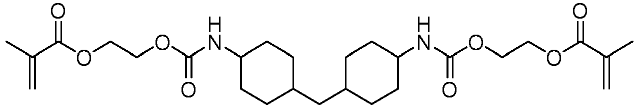

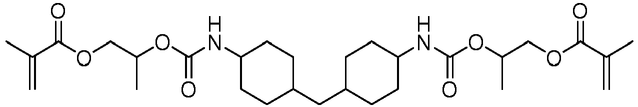

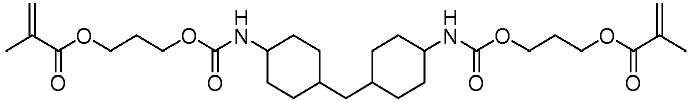

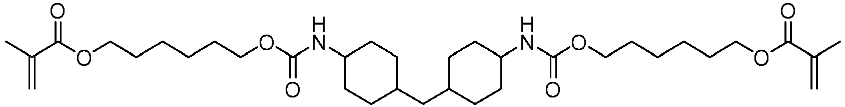

Most preferred monomers (a) are bis-[(2-methacryloyloxyethoxy-carbonyl)-amino]-2,2,4-trimethylhexane (UDMA) and V837, which is the reaction product of 2-isocyanatoethyl methacrylate with 2-hydroxyethyl methacrylate, and mixtures thereof.

The composites according to the invention preferably contain 2 to 30 wt.%, preferably 3 to 25 wt.% and more preferably 4 to 20 wt.% of one or more urethane di(meth)acrylates according to the formulas (IV), (V) or (VI). Unless otherwise stated, all weight percentages herein refer to the total weight of the composites.

The radically polymerizable material according to the present invention further comprise at least one **aromatic di(meth)acrylate monomer (b).**

Preferred monomers (b) are the urethane di(meth)acrylate derivatives of 1,3-bis(1-isocyanato-1-methylethyl)benzene disclosed in EP 0 934 926 A1. Particularly preferred is V380.

V380 is an urethane di(meth)acrylate derivative or mixture of urethane di(meth)acrylate derivatives according to the following formula:

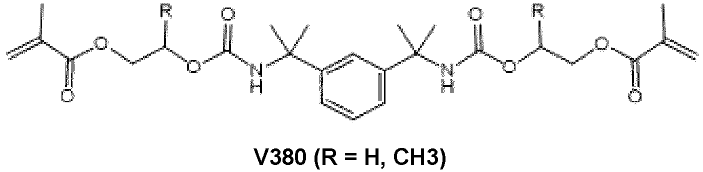

In the formula shown, the radicals R are each independently H or CH₃, where the radicals may have the same meaning or different meanings. Preferably, a random mixture is used which contains molecules in which both radicals are H, molecules in which both radicals are CH₃ and molecules in which one radical is H and the other radical is CH₃. The ratio of H to CH₃ in the mixture is preferably 7:3. Such a mixture is obtainable, for example, by reacting 1,3-bis(1-isocyanato-1-methylethyl)benzene with 2-hydroxypropyl methacrylate and 2-hydroxyethyl methacrylate.

Also preferred as monomers (b) are monomers which in addition to one or more aromatic groups comprise one or more urethane groups and. Particularly preferred monomers of this type are monomers according to **formula (VII):** 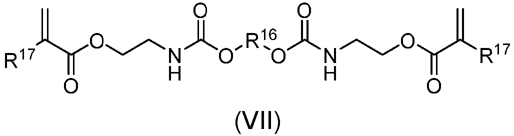 wherein
- R¹⁶: is a C₇-C₃₀-alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R¹⁸, where R¹⁸ is a branched or preferably linear C₁-C₂₀ alkyl group and/or acyl groups -C(O)-R¹⁹, where R¹⁹ is a branched or preferably linear C₁-C₂₀ alkyl group; and
- R¹⁷: is H or preferably CH₃.

Monomers according to formula (VII) can be synthesized via the reaction of a diol with 2-isocyanatoethyl methacrylate.

Particularly preferred monomers according to formula (VII) are:

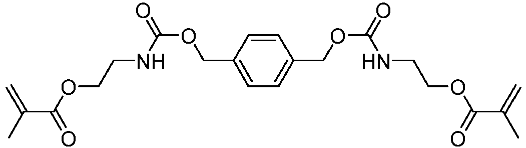

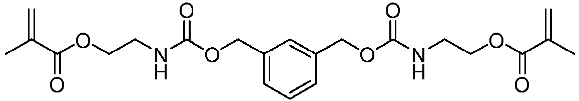

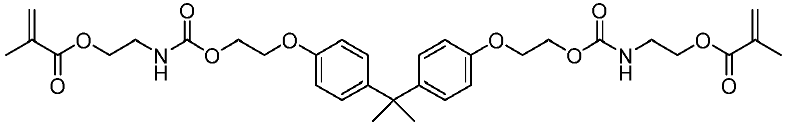

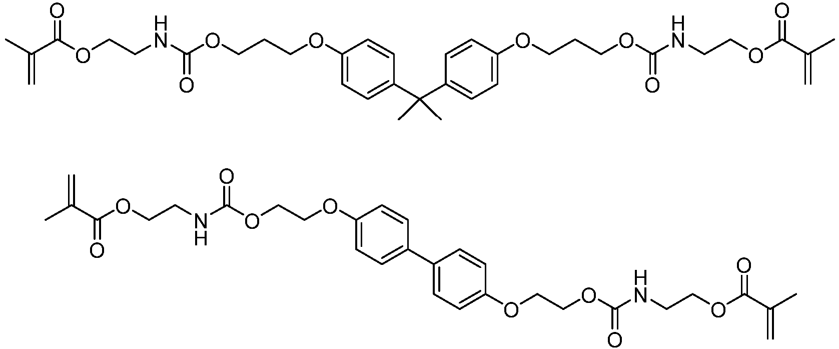

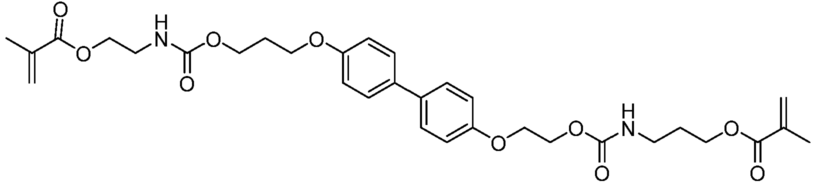

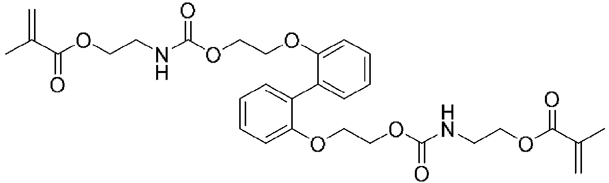

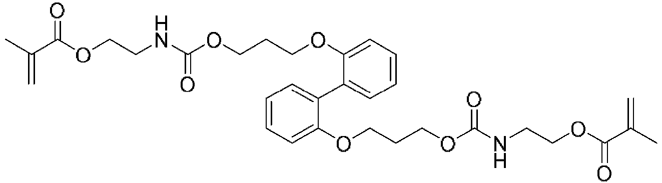

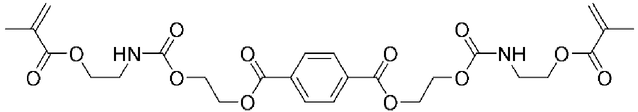

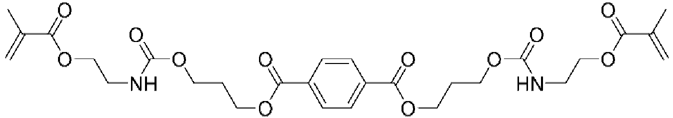

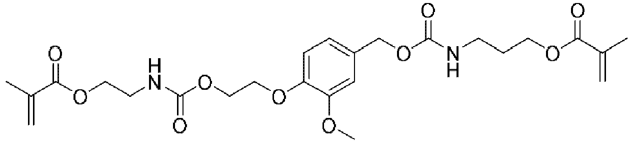

Further preferred aromatic di(meth)acrylate monomers (b) are hydroxyl group-bearing monomers according to **formula (VIII):** 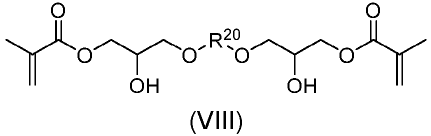 wherein
- R²⁰: is a C₆-C₃₀-aryl or alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R²¹, where R²¹ is a branched or preferably linear C₁-C₂₀ alkyl group, and/or acyl groups -C(O)-R²², where R²² is a branched or preferably linear C₁-C₂₀ alkyl group.

Particularly preferred monomers according to formula (VIII) are:

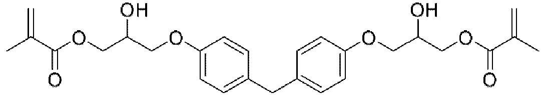

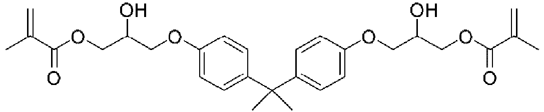

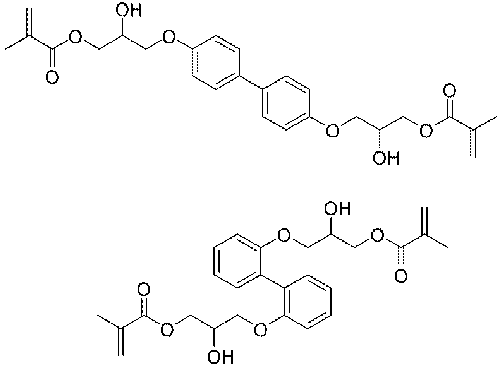

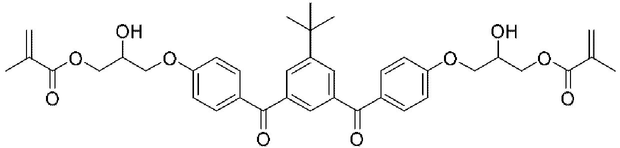

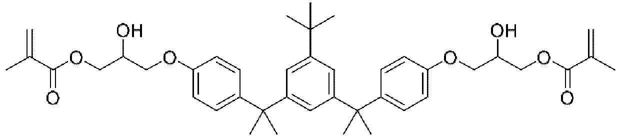

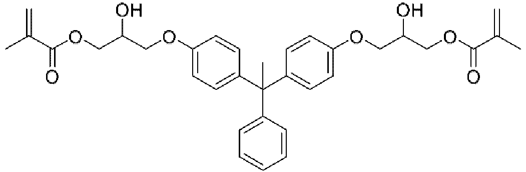

Most preferred monomers (b) are V380, in particular the V380 isomer 2-methyl-acrylic acid 2-[1-methyl-1-(3-{1-methyl-1-[2-(2-methyl-acryloyloxy)-ethoxycarbonylamino]-ethyl}-phenyl)-ethylcarbamoyloxy]propyl ester, 2,2-bis[4-(2-hydroxy-3-methacryloyl-oxypropoxy)-phenyl]propane (Bis-GMA), and mixtures thereof.

The composites according to the invention preferably contain 2 to 15 wt.% of one or more aromatic di(meth)acrylate (b).

### OPTIONAL MONOMERS

The dental materials according to the invention optionally contain one or more additional **dimethacrylate monomers (e)** that do not comprise hydroxy and/or urethane groups and are different from the monomers (a) and (b), preferably in an amount of 0 to 25 wt.%, more preferably 0 to 20 wt.%, and most preferably 2 to 20 wt.%. Preferred dimethacrylates are ethoxylated or propoxylated bisphenol A dimethacrylate, such as bisphenol A dimethacrylate with 3 ethoxy groups, which is available as SR-348c from Sartomer, or 2,2-bis[4-(2-methacryloyloxy-propoxy)phenyl]-propane, di-, tri- or tetraethylene glycol dimethacrylate, 1,4-butanediol dimethacrylate, 1,10-decanediol dimethacrylate (DsMA), polyethylene glycol or polypropylene glycol dimethacrylates, such as polyethylene glycol 200-dimethacrylate or polyethylene glycol 400-dimethacrylate (PEG-200- or PEG-400-DMA), or 1,12-dodecanediol dimethacrylate. Bisphenol A dimethacrylate SR-348c (Sartomer), PEG-400-DMA (NK ester 9G), and 1,10-decanediol dimethacrylate (DsMA) are particularly preferred with D₃MA being most preferred.

Furthermore, the dental materials according to the invention optionally contain one or more **monofunctional (meth)acrylate monomers (f)**, preferably in an amount of 0 to 25 wt.%, more preferably 0 to 20 wt.%, and most preferably 0 to 15 wt.%. Monofunctional (meth)acrylate monomers are monomers that comprise one radically polymerizable (meth)acrylate group, and accordingly difunctional (meth)acrylate monomers are monomers with two radically polymerizable (meth)acrylate groups. Monofunctional (meth)acrylate monomers are also called mono(meth)acrylate monomers or mono(meth)acrylates, and difunctional (meth)acrylate monomers are called di(meth)acrylate monomers or di(meth)acrylates. Preferred monofunctional monomers are furfuryl methacrylate, 2-phenoxyethyl methacrylate, 2-(o-biphenyl-oxy)ethyl methacrylate, 2-hydroxy-3-phenoxypropyl methacrylate, phenethyl methacrylate, 2-[(benzyloxycarbonyl)-amino]-ethyl methacrylate, 2-[(benzylcarbamoyl)oxy]-ethyl methacrylate, 1-phenoxypropan-2-yl methacrylate, 2-(p-cumylphenoxy)-ethyl methacrylate, tricyclodecane methacrylate, tricyclodecane methyl methacrylate and/or 2-(p-cumylphenoxy)ethyl methacrylate, and in particular cumylphenoxyethylene glycol methacrylate (CMP-1E).

### PHOTOINITIATOR SYSTEM

The composites according to the invention contain a **photoinitiator system (d).** Preferred photoinitiators are alpha-diketones derivatives, preferably camphorquinone (CQ), 9,10-phenanthrenequinone, 1-phenylpropane-1,2-dione, 2,2-dimethoxy-2-phenylacetophenone, diacetyl or 4,4'-dichlorobenzil or derivatives thereof. Camphorquinone (CQ), 2,2-dimethoxy-2-phenylacetophenone and mixtures thereof are most particularly preferred. Alpha-diketones are preferably used in combination a coinitiator. Bimolecular photoinitiators, i.e. photoinitiators that require a second component to reach their optimal activity, are referred to as Norrish type 2 photoinitiators. Preferred coinitiators are tertiary amines such as ethyl-4-(dimethylamino) benzoate, N,N-dimethylaminoethyl methacrylate, N,N-dimethyl-sym.-xylidine and triethanolamine. Other preferred coinitiators are N-phenyl glycine and its derivatives, 1,3-benzodioxole and its derivatives, tris(trimethylsilyl)silane, curcumin derivatives as well as sulfinate and sulfonate salts.

Iodonium salts can be added as third component in order to further increase the reactivity of the initiator systems. Preferred iodonium salts are bis(4-tert-butylphenyl)-iodonium hexafluorophosphate, bis(4-tert-butylphenyl)iodonium tetrafluoroborate, bis(4-tert-butylphenyl)iodonium nonafluoro-1-butanesulfonate, diphenyliodonium hexafluorophosphate, diphenyliodonium tetrafluoroborate, diphenyliodonium hexafluoroantimonate, diphenyliodonium 4-methylbenzenesulfonate, 4-octyloxydiphenyl-iodonium hexafluoroantimonate and diphenyliodonium trifluoromethanesulfonate.

Norrish type 1 photoinitiators can also be used. Norrish type 1 photoinitiators do not require a second component to reach their optimal activity. The materials according to the invention preferably contain at least one Norrish type 1 photoinitiator, more preferably a Norrish type 1 photoinitiator which is active in a wavelength range of from 400 to 500 nm.

Preferred Norrish type 1 photoinitiators are monoacyl- or bisacylphosphine oxides, di-acyldialkylgermanium and tetraacylgermanium compounds as well as tetraacylstan-nanes. Particularly preferred monomolecular photoinitiators are 2,4,6-trimethylbenzoyl diphenylphosphine oxide, bis(2,4,6-trimethylbenzoyl) phenylphosphine oxide, ethyl (2,4,6-trimethylbenzoyl) phenylphosphinate (TPO-L), dibenzoyldiethylgermane, bis(4-methoxybenzoyl)diethylgermanium (MBDEGe, trade name Ivocerin), tetrabenzoyl-germane, tetrakis(o-methylbenzoyl)germane, tetrakis(mesitoyl)stannane, and mixtures thereof.

Moreover, mixtures of the different photoinitiators can also be used, such as e.g. bis(4-methoxybenzoyl)diethylgermane or tetrakis(o-methylbenzoyl)germane in combination with camphorquinone and 4-dimethylaminobenzoic acid ethyl ester.

The compositions of the present invention preferably comprise 0.05 to 2.0 wt.%, more preferably 0.08 to 1.0 wt.%, and most preferably 0.1 to 0.8 wt.% of the photoinitiator system (d). The photoinitiator system comprises all components used to initiate the radical polymerization, in particular initiators and coinitiators.

### FILLERS

The compositions according to the invention contain at least one **inorganic glass filler** (c). Radiopaque glass fillers, such as barium or strontium aluminum silicate glasses and fluoroaluminosilicate (FAS) glass fillers, are preferred.

Preferred radiopaque glass fillers have the following composition (wt.%): SiO₂: 20-80; B₂O₃: 2-15, BaO or SrO: 10-40; Al₂O₃: 2-20; CaO and/or MgO: 0-20; Na₂O, K₂O, Cs₂O: 0-10 each; WOs: 0-20; ZnO: 0-20; La₂O₃: 0-10; ZrO₂: 0-15; P₂O₅: 0-30; Ta₂O₅, Nb₂O₅ or Yb₂O₃: 0-5; and CaF₂ and/or SrF₂ 0-10. Particularly preferred are radiopaque glass fillers having the composition (wt.%): SiO₂: 40-75; B₂O₃: 2-15; BaO or SrO: 15-35; Al₂O₃: 2-15; CaO and/or MgO: 0-10; and Na₂O: 0-10.

Preferred fluoroaluminosilicate (FAS) glass fillers have the following composition (wt.%): SiO₂: 20-35; Al₂O₃: 15-35; BaO or SrO: 10-25; CaO: 0-20; ZnO: 0-15; P₂O₅: 5-20; Na₂O, K₂O, Cs₂O: 0-10 each; and CaF₂: 0.5-20. Particularly preferred are FAS fillers with the composition (wt.%): SiO₂: 20-30; Al₂O₃: 20-30; BaO or SrO: 10-25; CaO: 5-20; P₂O₅: 5-20; Na₂O: 0-10; and CaF₂: 5-20.

The weight percentages given for the radiopaque glass fillers and fluoroaluminosilicate glass fillers refer to the total weight of the glasses and, with the exception of fluorides, are calculated on the basis of the oxides.

The inorganic glass filler (c) preferably has an average particle size of 0.15 to 1.5 µm and particularly preferably of 0.15 to 1.0 µm.

The compositions of the present invention preferably comprise from 30 to 90 wt.%, more preferably 35 to 85 wt.%, and most preferably 40 to 80 wt.%, of the at least one inorganic filler (c).

The dental materials according to the invention can optionally comprise 0 to 30 wt.%, preferably 0 to 25 wt.%, and more preferably 3 to 20 wt.%, of at least one **mixed oxide filler (h).** Particularly preferred fillers are mixed oxides of SiO₂ and ZrO₂, with a particle size between 10 and 300 nm.

Furthermore, the dental materials according to the invention can optionally contain 0 to 30 wt.%, preferably 3 to 25 wt.%, and more preferably 5 to 20 wt.%, of one or more **additional fillers (i).** Preferred additional fillers are quartz, pyrogenic silica, composite fillers, and Ytterbium trifluoride.

Composite fillers are also referred to as isofillers. These are fragmentary polymers which in turn contain an inorganic filler, preferably pyrogenic SiO₂ and/or ytterbium trifluoride. Preferred are polymers based on dimethacrylates. For the production of isofillers, the inorganic filler(s) is/are incorporated, for example, into a dimethacrylate resin matrix, and the resulting composite paste is subsequently thermally polymerized and then ground. A composite filler preferred according to the invention can be prepared, for example, by thermally curing a mixture of Bis-GMA (8.80% by weight), UDMA (6.60% by weight), 1,10-decanediol dimethacrylate (5.93% by weight), dibenzoyl peroxide + 2,6-di-tert. butyl-4-methylphenol (together 0.67 wt.%), glass filler (average grain size 0.4 µm; 53.0 wt.%) and YbFs (25.0 wt.%) and subsequent grinding of the cured material to the desired grain size. Another composite filler preferred according to the invention is a hot-cured composite filler ground to the desired grain size and prepared from UDMA (23.58 % by weight), 1,10-decanediol dimethacrylate (5.92 % by weight), dibenzoyl peroxide (0.50 % by weight), fumed silica Aerosil OX50 (50.0 % by weight) and YbFs (20.0 % by weight). All percentages refer to the total mass of the composite filler.

The fillers (h) and (i) are preferably also surface modified, preferably with a silane and more preferably with a silane according to formula (I).

Unless otherwise stated, all particle sizes are weight-average particle sizes [D₅₀], wherein the particle-size determination in the range of from 0.1 µm to 10 µm is measured by means of static light scattering, preferably using an LA-960 static laser scattering particle size analyzer (Horiba, Japan). Here, a laser diode with a wavelength of 655 nm and an LED with a wavelength of 405 nm are used as light sources. The use of two light sources with different wavelengths makes it possible to measure the entire particle-size distribution of a sample in only one measurement pass, wherein the measurement is carried out as a wet measurement. For this, a 0.1 to 0.5% aqueous dispersion of the filler is prepared, and the scattered light thereof is measured in a flow cell. The scattered-light analysis for calculating particle size and particle-size distribution is performed in accordance with the Mie theory according to DIN/ISO 13320: 2020. Particle sizes smaller than 0.1 µm are preferably determined by means of dynamic light scattering (DLS). The measurement of the particle size in the range of from 5 nm to 0.1 µm is preferably effected by dynamic light scattering (DLS) of aqueous par-ticle dispersions, preferably with a Malvern Zetasizer Nano ZS (Malvern Instruments, Malvern UK) with a He-Ne laser with a wavelength of 633 nm, at a scattering angle of 90° at 25° C. Particle sizes smaller than 0.1 µm can also be determined by means of SEM or TEM spectroscopy. The transmission electron microscopy (TEM) is preferably carried out with a Philips CM30 TEM at an accelerating voltage of 300 kV. For the preparation of the samples, drops of the particle dispersion are applied to a 50 Å thick copper grid (mesh size 300), which is coated with carbon, and then the solvent is evaporated.

The polymerizable compositions according to the invention preferably do not contain inorganic fillers having an average particles size (D50) of greater than 1.5 µm.

### SILANES

According to the present invention, the inorganic filler (c) is silanized with a silane according to the formula (I). Preferably the fillers (h) and (i) are also silanized with a silane according to formula (I), if present.

In formula (I), the residue R³ may be a group according to formula II or III. Silanes in which R³ is a group according to formula (II) can be represented by the following formula Ia, in which the variables have the meanings defined above:

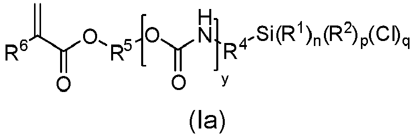

Particularly preferred silanes according to **formula (Ia)** are:

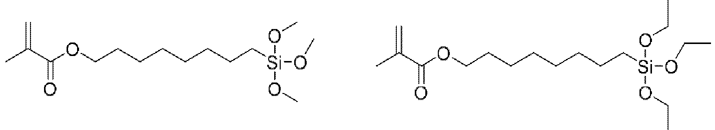

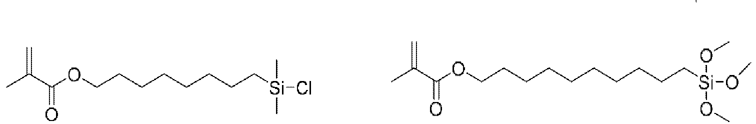

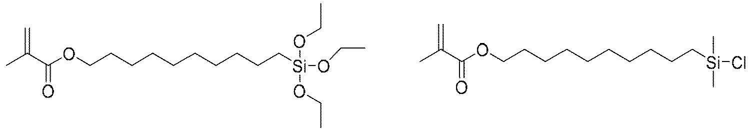

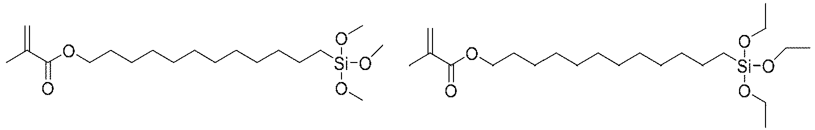

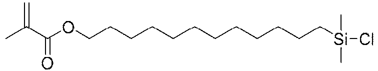

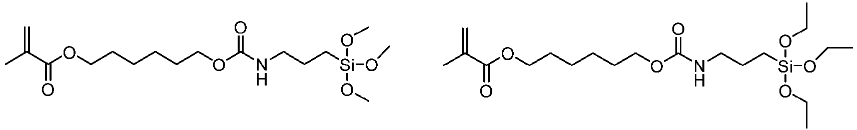

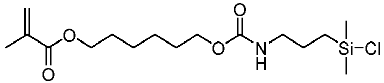

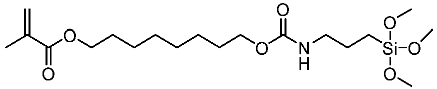

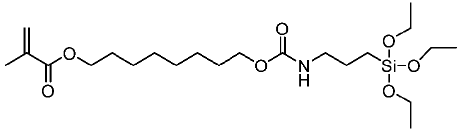

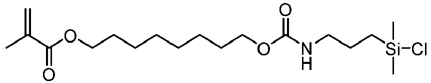

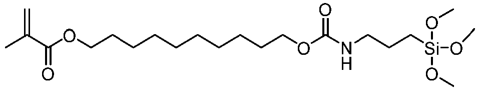

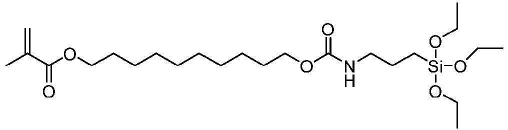

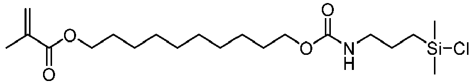

Silanes in which R³ is a group according to formula (III) can be represented by the following **formula (Ib),** in which the variables have the meanings defined above:

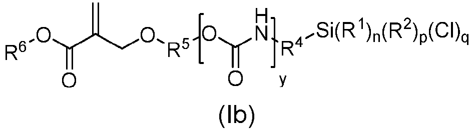

Particularly preferred silanes according to formula (Ib) are:

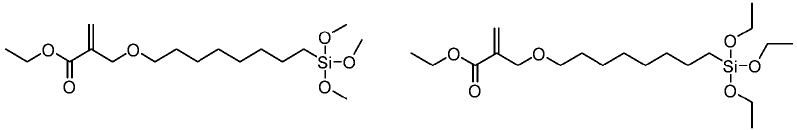

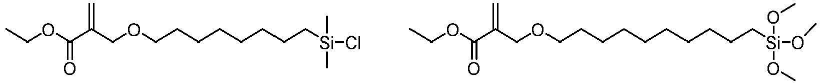

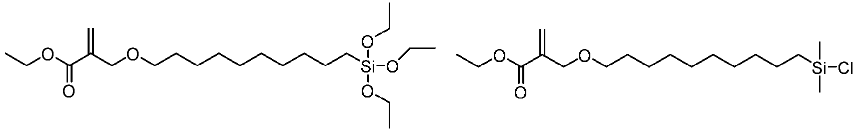

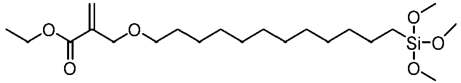

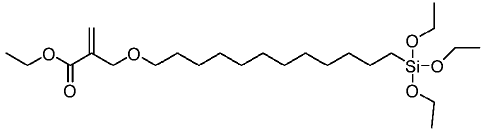

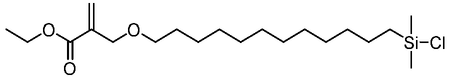

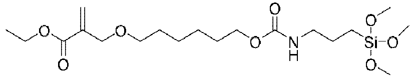

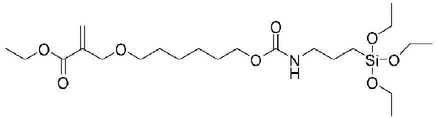

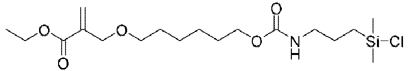

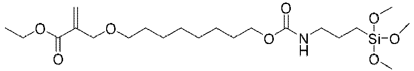

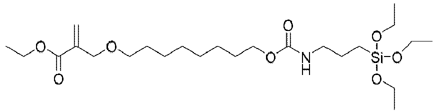

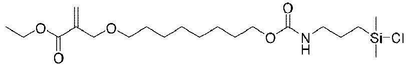

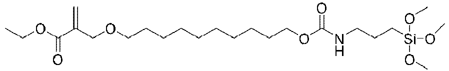

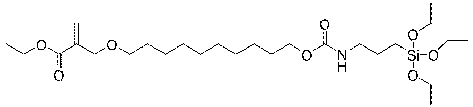

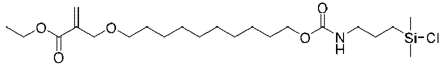

The filler particles according to the invention are preferably functionalized with 0.1 to 15 wt.% of one or more silanes Ia and/or Ib. More preferably the filler particles are functionalized with 0.2 to 10 wt.% and most preferably with 0.5 to 5 wt.% of one or more silanes Ia and/or Ib. These weight percentages refer to the weight of the filler. The functionalization process can be conducted without solvents in presence of traces of water or in a dispersion in organic solvents. Suitable organic solvents include, but are not limited to, cyclohexane, dioxane, dichloromethane, THF, chloroform, acetone, DMSO and DMF. In particular aprotic solvents are preferred. The silanization is preferably carried out in presence of a catalyst such as an primary amine. More preferably the functionalization is carried out in cyclohexane in presence of the base n-propyl amine.

### CHAIN TRANSFER AGENTS

According to a preferred embodiment, the compositions according to the invention optionally contain a chain transfer agent (CTA) (g). By the addition of a CTA, the shrinkage stress of dimethacrylate-based compositions can be reduced during photopolymerization. It was surprisingly also found that the addition of a CTA can increase the desired drop in consistency when the temperature is increased.

Preferred CTAs are allyl sulfones and allyl sulfides according to **formula (IX):** 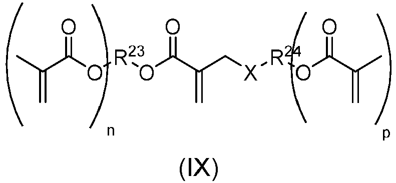 wherein
- X: is SO₂ and
- R²³: is a branched or preferably linear C₁-C₁₆, preferably C₁-C₁₄ and more preferably C₁-C₁₂ alkyl group that can be interrupted by one to three, preferably one to two O atoms,
- R²⁴: is a tolyl group (in this case p = 0) or a branched or preferably linear C₁-C₁₆, preferably C₁-C₁₄ and more preferably C₁-C₁₀ alkyl group that can be interrupted by one to three, preferably one to two O atoms and that can also be interrupted by either an ester group (-(C=O)-O-) or a urethane group (-O(C=O)-NH-) and that can be functionalized by one or two OH groups,
- n: is 0 or 1, and
- p: is 0 or 1;
or
- X: is S and
- R²³: is a branched or preferably linear C₁-C₁₀, preferably C₁-C₆ and more preferably C₁-C₄ alkyl group,
- R²⁴: is a branched or preferably linear C₁-C₁₆, preferably C₁-C₁₄ and more preferably C₁-C₁₀ alkyl group that can be interrupted by one to three, preferably one to two O atoms and that can also be interrupted by either a -(C=O)-O- ester group or a -O(C=O)-NH- urethane group and that can be functionalized by one or two OH groups,
- n: is 0 or 1, and
- p: is 0 or 1.

Particularly preferred chain transfer agents according to formula (IX) are:

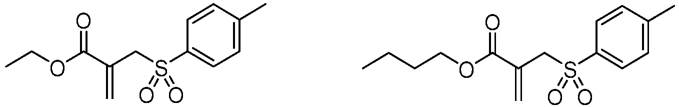

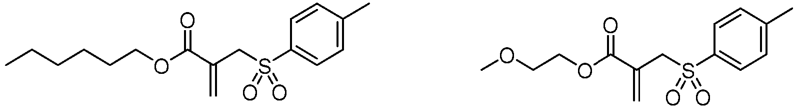

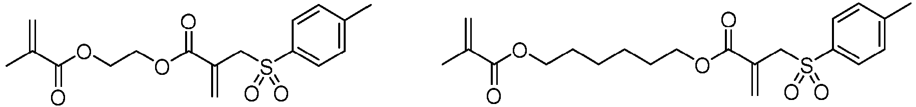

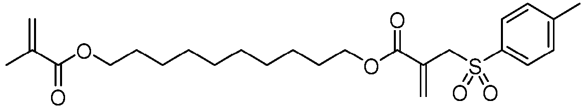

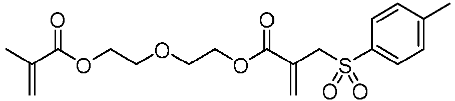

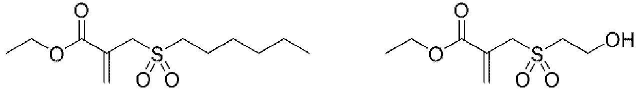

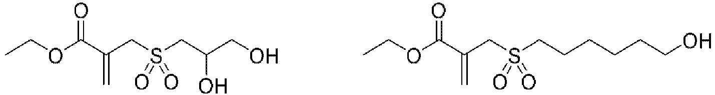

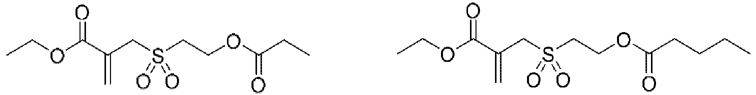

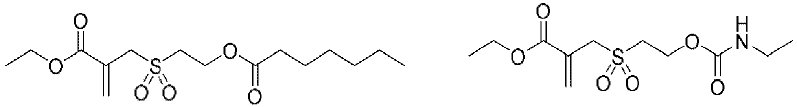

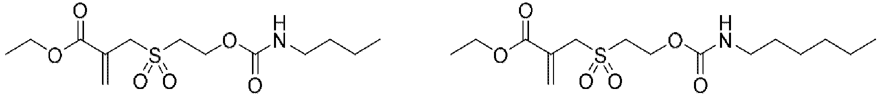

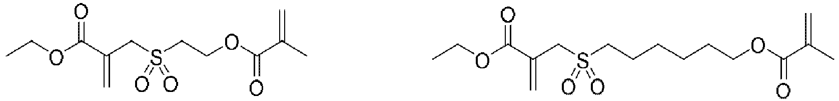

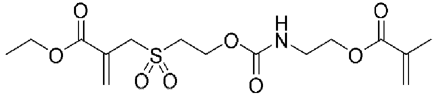

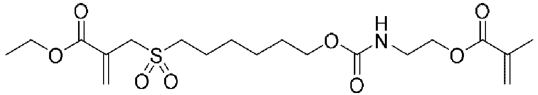

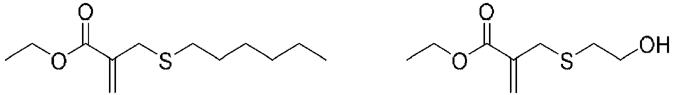

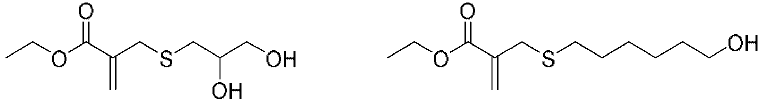

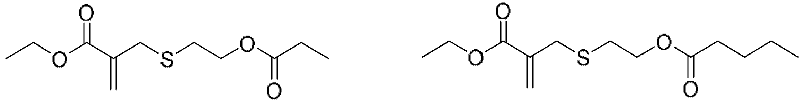

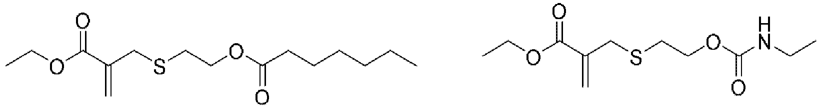

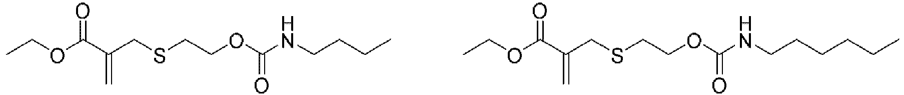

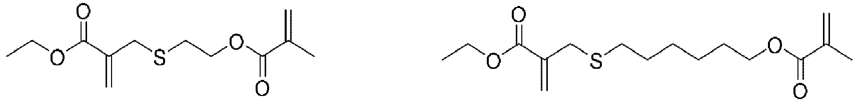

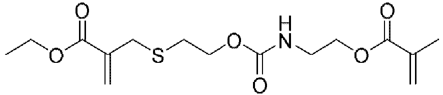

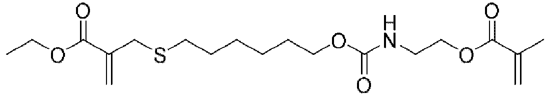

The composites according to the invention preferably contain 0.3 to 10 wt.%, more preferably 0.4 to 7 wt.% and most preferably 0.5 to 5 wt.% of one or more chain transfer reagents (g).

### ADDITIVES

The compositions according to the invention can additionally contain one or more additives, preferably one or more stabilizers, such as e.g. polymerization stabilizers, pigments, optical brighteners, fluorescent agents, plasticizers, and/or UV absorbers.

The compositions according to the invention preferably comprise at least one stabilizer, preferably MEHQ, BHT, TEMPO or a mixture thereof.

The composites according to the invention preferably contain up to 0.1 wt.%, more preferably up to 0.05 wt.% and most preferably 0.01 to 0.10 wt.% or 0.01 to 0.05 wt.% of one or more additives.

The radically polymerizable compositions according to the present invention preferably comprise
(a) 2 to 34 wt.%, more preferably, 3 to 25 wt.%, and most preferably 4 to 20 wt.% of the at least one urethane di(meth)acrylate monomer;
(b) 3 to 34 wt.%, more preferably, 4 to 25 wt.%, and most preferably 5 to 20 wt.% of the at least one aromatic di(meth)acrylate monomer;
(c) 30 to 90 wt.% , more preferably, 35 to 85 wt.%, and most preferably 40 to 80 wt.% of the at least one glass filler that is at least partially functionalized with a silane according to the formula (I); and
(d) 0.05 to 2.0 wt.%, more preferably, 0.08 to 1.0 wt.%, and most preferably 0.1 to 0.8 wt.% of the at least one photoinitiator system for the radical polymerization,
based on the total weight of the composition.

Preferably, the radically polymerizable compositions of the present invention additionally comprise:
(e) 0 to 25 wt.%, more preferably, 0 to 20 wt.%, and most preferably 2 to 20 wt.% of at least one dimethacrylate monomer that is different to monomers (a) and (b); and/or
(f) 0 to 25 wt.% , more preferably, 0 to 20 wt.%, and most preferably 2 to 20 wt.% of at least one monofunctional (meth)acrylate monomer; and/or
(g) 0.3 to 10 wt.% , more preferably, 0.4 to 7 wt.%, and most preferably 0.5 to 5 wt.% of at least one chain transfer agent, preferably an allyl sulfide or an allyl sulfone; and/or
(h) 0 to 30 wt.% , more preferably, 0 to 25 wt.%, and most preferably 3 to 20 wt.% of at least one mixed oxide filler; and/or
(i) 0 to 30 wt.% , more preferably, 3 to 25 wt.%, and most preferably 5 to 20 wt.% of one or more additional fillers.

At room temperature, the compositions of the present invention have a consistency like a typical packable dental composite. The consistency is determined using a texture analyzer as described in the examples. Dental materials are packable if they have a consistency (absolute positive force) of ≥ 500 g. When heated, the consistency of the composites decreases and it was surprisingly found that the compositions of the present invention, unlike commercially available dental composites, have a consistency of ≤ 60 g (absolute positive force) at 60°C, which is typical for a flowable composite. At about 60°C, the materials therefore behave like a conventional flowable composite, they flow with minimal force and can be dispensed through a needle tip. They can adapt to the shape of the prepared cavity and flow into undercuts. When cooled to about 30°C, i.e. the application temperature in the mouth, their consistency changes and they behave like a packable dental composite. They are non-sticky and moldable, allowing the dentist to sculpt the natural anatomy of the tooth before curing, i.e. to shape the occlusal surfaces and the interproximal walls. The materials of the present invention thus make it possible to restore the tooth with a single material, which is a considerable advantage.

It is assumed that the silanes of formula (I) are able to interact with the monomers via weak interactions such as hydrogen bonds and that these interactions are weakened when the temperature is increased, leading to the desired decease in viscosity. The silanes of formula (I) are characterized by a relatively long spacer group between the Si atom and the methacrylate group, which presumably favors the desired change in consistency. The spacer comprises at least eight carbon atoms. The interaction of the spacer with the monomers and the influence of the temperature on the consistency can be modified by incorporating a urethane group into the spacer group. According to one embodiment, the present invention relates to fillers modified with a silane according to formula (I) wherein the spacer comprises a urethane group (y = 1) and according to another embodiment to fillers modified with a silane without a urethane group (y = 0). Compositions comprising a filler that is modified with a silane without urethane groups are preferred.

The composites of the present invention comprise a relatively large amount of filler (c) with a relatively small particle size. Due to the small particle size, the filler (c) has a relatively large surface area which can bind a relatively large amount of the silane, which then interacts with the monomer components of the composites. It is assumed that this is a major reason for the desired temperature dependence of the consistency of the materials. By varying the particle size and the type of silane, the flow properties can be fine-tuned and adapted to the desired monomer mixture.

The radically polymerizable compositions of the present invention are particularly suitable as dental materials, especially as dental filling materials for restoring damaged teeth. They are also particularly suitable for use in a method for restoring teeth damaged by caries or similar diseases. The compositions of the present invention can also be used in a non-therapeutic manner, for instance for the extra-oral restoration of dentures or artificial teeth.

For use, the tooth or teeth to be treated are prepared by the dentist and then the heated composition is placed in the prepared cavity. The cooled material is then sculpted and shaped and finally hardened.

The invention is explained in more detail in the following with reference to examples.

### EXAMPLES

### Example 1:

### Synthesis of the urea silane MUATS

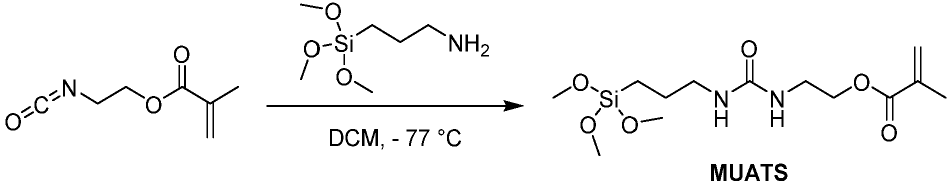

Distilled 3-aminopropyltrimethoxysilane (11.56 g, 64.45 mmol) was dissolved in dry dichloromethane (DCM) (30 mL) under an argon atmosphere. The solution was cooled down to -58 °C using a dry ice/isopropanol bath. 2-Isocyanatoethyl methacrylate (IEMA) (10.00 g, 64.45 mmol, 1.0 equiv.) was added slowly dropwise to the reaction mixture, due to the exothermicity of the reaction. The reaction was traced by IR and NMR spectroscopy till full conversion was reached. The solvent was evaporated, while dry air was bubbled through the flask. The residual colorless oil was characterized by NMR spectroscopy. The desired product (21.55 g) was obtained as viscous colorless oil in quantitative yield.

¹H NMR (CDCl₃, 400 MHz, δ): 6.15 - 6.08 (m, 1H, =CH₂); 5.62 - 5.56 (m, 1H, =CH); 5.55 - 5.44 (m, 1H, NH); 5.44 - 5.32 (m, 1H, NH); 4.20 (t, J = 5.6 Hz, 2H, OCH₂); 3.56 (s, 9H, OCH₃); 3.47 (q, J = 5.5 Hz, 2H, NCH₂); 3.14 (q, J = 6.2 Hz, 2H, NCH₂); 1.94 (s, 3H, CH₃); 1.59 (qt, 7.8 Hz, 2H, CH₂); 0.64 (t, J = 8.3 Hz, 2H, SiCH₂). ¹³C NMR (CDCl₃, 100.6 MHz, δ): 167.5 (C=O); 158.5 (C=O); 136.1 (=C); 126.0 (=CH₂); 64.3 (OCH₂); 50.6 (SiOCH₃); 42.9 (NCH₂); 39.4 (NCH₂); 23.5 (CH₂); 18.3 (CH₃); 6.4 (SiCH₂).

### Example 2

### Synthesis of the urethane silane MUETS

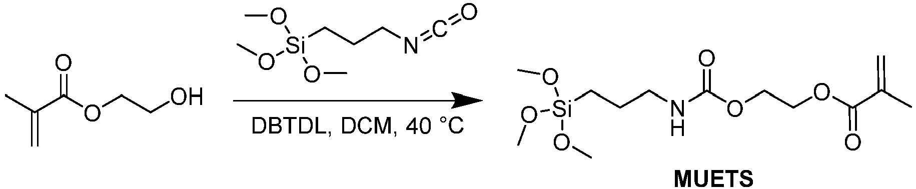

2-Hydroxyethyl methacrylate (HEMA) (8.00 g, 61.47 mmol) and a catalytic amount of dibutyltin dilaurate (23 mg) were dissolved in dry dichloromethane (DCM) (4 mL). The solution was heated up to 40 °C and 3-isocyanatopropyltrimethoxysilane (dist.) (12.26 g, 61.47 mmol, 1.0 equiv.) was added dropwise (10 min). The resulting solution was stirred at 40 °C under nitrogen atmosphere for 3.5 h. The reaction was traced by IR and NMR spectroscopy until full conversion was reached. The solvent was evaporated, while dry air was bubbled through the flask. The residual colorless oil was characterized by NMR spectroscopy. The desired product (20.62 g) was obtained as viscous colorless oil in quantitative yield.

¹H NMR (CDCl₃, 400 MHz, δ): 6.16 - 6.09 (m, 1H, =CH₂); 5.62 - 5.55 (m, 1H, =CH); 4.96 (bs, 1H, NH); 4.38 - 4.27 (m, 4H, OCH₂); 3.57 (s, 9H, OCH₃); 3.14 (q, J = 6.4 Hz, 2H, NCH₂); 1.95 (s, 3H, CH₃); 1.62 (qt, 7.9 Hz, 2H, CH₂); 0.64 (t, J = 8.4 Hz, 2H, SiCH₂). ¹³C NMR (CDCl₃, 100.6 MHz, δ): 167.3 (C=O); 156.2 (C=O); 136.1 (=C); 126.1 (=CH₂); 63.1 (OCH₂); 62.6 (OCH₂); 50.7 (SiOCH₃); 43.5 (NCH₂); 23.2 (CH₂); 18.4 (CH₃); 6.4 (SiCH₂).

### Example 3:

### Synthesis of the urethane silane SI-1

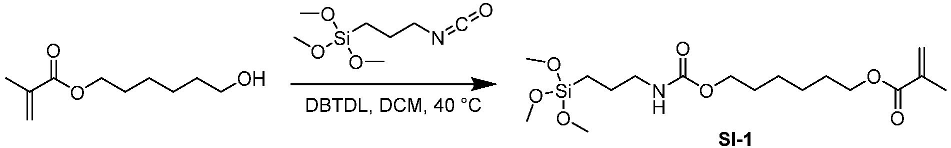

6-Hydroxyhexyl methacrylate (17.30 g, 92.89 mmol) and a catalytic amount of dibutyltin dilaurate (DBTDL) (35 mg) were dissolved in dry dichloromethane (45 mL). The solution was heated up to 40°C and distilled 3-isocyanatopropyltrimethoxysilane (19.07 g, 92.89 mmol, 1.0 equiv.) was added dropwise (10 min). The resulting solution was stirred at 40°C under nitrogen atmosphere for 3.5 h. The reaction was traced by IR and NMR spectroscopy until full conversion was reached. The solvent was removed under reduced pressure. The residual colorless oil was characterized by NMR spectroscopy. The desired product (33.39 g) was obtained as viscous colorless oil in 92 % yield.

¹H NMR (CDCl₃, 400 MHz, δ): 6.12 - 6.06 (m, 1H, =CH₂); 5.58 - 5.52 (m, 1H, =CH); 5.04 (bs, 1H, NH); 4.14 (t, J = 6.6 Hz, 2H, OCH₂); 4.04 (t, J = 6.6 Hz, 2H, OCH₂); 3.57 (s, 9H, OCH₃); 3.16 (q, J = 6.6 Hz, 2H, NCH₂); 1.94 (s, 3H, CH₃); 1.75 -1.51 (m, 6H, CH₂); 1.48 -1.32 (m, 4H, CH₂); 0.64 (t, J = 8.3 Hz, 2H, SiCH₂). ¹³C NMR (CDCl₃, 100.6 MHz, δ): 167.6 (C=O); 156.8 (C=O); 136.6 (=C); 125.3 (=CH₂); 64.7 (2x OCH₂); 50.7 (SiOCHs); 43.4 (NCH₂); 29.0 (CH₂); 28.6 (CH₂); 25.8 (CH₂); 25.6 (CH₂); 23.3 (CH₂); 18.4 (CH₃); 6.4 (SiCH₂).

### Example 4:

### Synthesis of the urethane silane SI-2

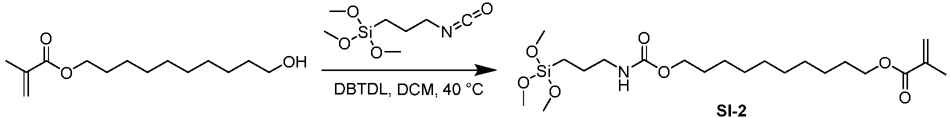

10-Hydroxydecyl methacrylate (19.69 g, 81.24 mmol) and a catalytic amount of dibutyltin dilaurate (DBTDL) (31 mg) were dissolved in dry dichloromethane (37 mL). The solution was heated up to 40°C and 3-isocyanatopropyltrimethoxysilane (dist.) (16.68 g, 81.24 mmol, 1.0 equiv.) was added dropwise (10 min). The resulting solution was stirred at 40°C under nitrogen atmosphere for 3.5 h. The reaction was traced by IR and NMR spectroscopy until full conversion was reached. The solvent was removed under reduced pressure. The residual colorless oil was characterized by NMR spectroscopy. The desired product (33.76 g) was obtained as viscous colorless oil in 93 % yield.

¹H NMR (CDCl₃, 400 MHz, δ): 6.12 - 6.06 (m, 1H, =CH₂); 5.58 - 5.52 (m, 1H, =CH); 5.00 (bs, 1H, NH); 4.14 (t, J = 6.7 Hz, 2H, OCH₂); 4.03 (t, J = 6.8 Hz, 2H, OCH₂); 3.57 (s, 9H, OCH₃); 3.16 (q, J = 6.5 Hz, 2H, NCH₂); 1.94 (s, 3H, CH₃); 1.73 -1.50 (m, 6H, CH₂); 1.42 -1.21 (m, 12H, CH₂); 0.64 (t, J = 8.2 Hz, 2H, SiCH₂). ¹³C NMR (CDCl₃, 100.6 MHz, δ): 167.5 (C=O); 156.8 (C=O); 136.5 (=C); 125.1 (=CH₂); 64.8 (2x OCH₂); 50.5 (SiOCHs); 43.3 (NCH₂); 29.4 (2x CH₂); 29.3 (CH₂); 29.2 (CH₂); 29.1 (CH₂); 28.6 (CH₂); 26.0 (CH₂); 25.8 (CH₂); 23.2 (CH₂); 18.4 (CH₃); 6.3 (SiCH₂).

### Example 5:

### Functionalization of 1.0 µm glass particles with SI-1 and SI-2

A dental barium-aluminum-borosilicate glass powder having an average particles size of D₅₀ = 1.0 µm (GM27884; Schott AG) was silanized with the commercially available silanes 3-methacryloxypropyl trimethoxysilane (MPTS), 8-methacryloxyoctyl trimethoxysilane (MOTS) as well as 3,3-dimethoxy-8-oxo-2-oxa-7,9-diaza-3-silaundecan-11-yl methacrylate (MUATS; example 1), 3,3-dimethoxy-8-oxo-2,9-dioxa-7-aza-3-silaundecan-11-yl methacrylate (MUETS; example 2) and the silanes SI-1 and SI-2 of examples 3 and 4.

The glass powder (50 g) was dispersed in cyclohexane (128 mL) using a magnetic stirrer at room temperature under air for 30 min. The respective silane (2.01 mmol; 0.50 g MPTS, 0.64 g MOTS, 0.68 g MUATS, 0.68 g MUETS, 0.79 g SI-1 or 0.90 g SI-2) and n-propylamine (0.10 g, 1.69 mmol, 0.8 equiv.) were added dropwise. The resulting suspension was stirred at room temperature for 24 h. Subsequently, the solvent was evaporated under reduced pressure. The residue was sieved by using a 200 µm sieve, dried in an oven at 50°C for 5 days, washed with cyclohexane (3 x), centrifugated (5000 rpm, 5 min), dried in an oven at 50°C for 2 days and sieved by using a 90 µm sieve.

### Example 6:

### Functionalization of 0.7 µm glass particles

A dental barium-aluminum-borosilicate glass powder having an average particles size of D₅₀ = 0.7 µm (GM27884; Schott AG) was silanized with the commercially available silanes 3-methacryloxypropyl trimethoxysilane (MPTS) and 8-methacryloxyoctyl trimethoxysilane (MOTS).

The glass powder (100 g) was dispersed in cyclohexane (256 mL) using a magnetic stirrer at room temperature under air for 30 min. The selected silane (5.72 mmol; 1.42 g MPTS, 1.82 g MOTS) and n-propylamine (0.20 g, 3.38 mmol, 0.6 equiv.) were added dropwise. The resulting suspension was stirred at room temperature for 24 h. Subsequently, the solvent was evaporated under reduced pressure. The residue was sieved by using a 200 µm sieve, dried in an oven at 50 °C for 5 days, washed with cyclohexane (3 x), centrifugated (5000 rpm, 5 min), dried in an oven at 50 °C for 2 days and sieved by using a 90 µm sieve.

### Example 7:

### Functionalization of 3.0 my glass particles with 3-methacryloxypropyl trimethoxysilane (MPTS)

A dental barium-aluminum-borosilicate glass powder having an average particles size of D₅₀ = 3.0 µm (GM27884; Schott AG) was silanized with the commercially available silane 3-methacryloxypropyl trimethoxysilane (MPTS).

The glass powder (100 g) was dispersed in cyclohexane (256 mL) using a magnetic stirrer at room temperature under air for 30 min. MPTS (1.69 mmol; 0.42 g) and n-propylamine (0.08 g, 1.35 mmol, 0.8 equiv.) were added dropwise. The resulting suspension was stirred at room temperature for 24 h. Subsequently, the solvent was evaporated under reduced pressure. The residue was sieved by using a 200 µm sieve, dried in an oven at 50 °C for 5 days, washed with cyclohexane (3 x), centrifugated (5000 rpm, 5 min), dried in an oven at 50 °C for 2 days and sieved by using a 90 µm sieve.

### Example 8:

### Formulation of packable dental composites C1-C10

The packable dental composites **C1-10** described in Table 2 were obtained by mixing the monomer mixtures **M_{Stock 1}, M_{CTA}, M_{Stock 2}** (Table 1) and the functionalized barium-aluminum-borosilicate glass powders of Examples 5 to 7. 2,2-Bis-[4-(2-hydroxy-3-methacryloyloxypropoxy)phenyl]propane (Bis-GMA), 1,6-bis-[2-methacryloyloxy-ethoxycarbonylamino]-2,4,4-trimethylhexane (UDMA), decanediol-1,10-dimeth-acrylate (DsMA) and triethylene glycol dimethacrylate (TEGDMA) were used as monomers. Combinations of camphorquinone (CQ)/ ethyl-4-dimethylamino benzoate (EDAB) and bis(4-methoxybenzoyl)diethylgermanium (Ivocerin) were used as photoinitiator system and hydroquinone monomethyl ether (MEHQ) or butylated hydroxytoluene (BHT) was added as stabilizer. 2-Ethyl-2-(tosylmethyl)acrylate (ASEE) was added as chain transfer agent (CTA) in M_{CTA}. The phases were homogenized by using the kneading machine Linden LPM 01 (1 h, three times under ambient conditions) and deaerated (10 min, 3 times at 200 mbar). The filler content of each packable composite was increased until a consistency of 2800 to 3400 g was measured at 23°C using the texture analyzer.

**Table 1: Composition of monomer mixtures M_{Stock 1}, M_{CTA}, M_{Stock 2}**

| **Monomer mixture** | **Mstock ₁ (wt.%)** | **M_{CTA} (wt.%)** | **M_{Stock 2} (wt.%)** |
|---|---|---|---|
| Bis-GMA | 39.51 | 37.51 | 62.56 |
| UDMA | 39.51 | 37.51 | 21.82 |
| D₃MA | 19.75 | 18.75 | - |
| TEGDMA | - | - | 14.88 |
| ASEE | - | 5.00 | - |
| Camphorquinone | 0.33 | 0.33 | 0.23 |
| EDAB | 0.66 | 0.60 | 0.40 |
| Ivocerin^{®} | 0.25 | 0.25 | - |
| MEHQ | 0.05 | 0.05 | - |
| BHT | - | - | 0.11 |

### Example 9:

### Flexural strength and flexural modulus of hardened composites C1-C10

To obtain flexural strength specimens (2 × 2 × 25 mm), the composites C1-C10 were pressed in a steel mold, and irradiated by using a LED cube 100 (channel 1 (λ = 460 nm) + channel 2 (λ = 410 nm) at 60 % intensity, Hönle) controlled by a LED Powerdrive (Hönle) for 2*40 s. The cured samples were stored dry at room temperature or in distilled water at 37°C for 24 h before the determination of the mechanical properties. For the evaluation of the mechanical properties, three-point bending tests (span: 20 mm) with a speed of 0.8 mm min⁻¹ using a Z2.5/TS universal testing machine (Zwick, Germany) were performed according to ISO 4049. The results are shown in Table 3.

The cured composites containing glass filler functionalized with a silane according to formula I (MOTS, SI-1 and SI-2) showed excellent flexural strength in comparison to the reference composite C1 containing glass filler functionalized with MPTS which is the most commonly used silane in dental composites. Due to higher filler loading at similar consistency, MOTS, SI-1, and SI-2 based systems presented improved flexural modulus values after storage in water in comparison to the reference materials.

**Table 2: Composition of the packable composites C1-C10**

| **Component** | **Composite [wt.%]** | | | | | | | | | |
|---|---|---|---|---|---|---|---|---|---|---|
| | **C1*)** | **C2** | **C3*)** | **C4*)** | **C5** | **C6** | **C7** | **C8*)** | **C9** | **C10*)** |
| M_{Stock 1} | 29.10 | 24.95 | 30.25 | 29.00 | 24.00 | 23.25 | - | - | - | - |
| M_{CTA} | - | - | - | - | - | - | 24.75 | - | - | - |
| M_{Stock 2} | - | - | - | - | - | - | - | 29.50 | 25.85 | 20.70 |
| 0.7 µm glass powder sil MPTS**) | - | - | - | - | - | - | - | 70.50 | - | - |
| 1.0 µm glass powder sil MPTS**) | 70.90 | - | - | - | - | - | - | - | - | - |
| 3.0 µm glass powder sil MPTS**) | - | - | - | - | - | - | - | - | - | 79.30 |
| 0.7 µm glass powder sil MOTS | - | - | - | - | - | - | - | - | 74.15 | - |
| 1.0 µm glass powder sil MOTS | - | 75.05 | - | - | - | - | 75.25 | - | - | - |
| 1.0 µm glass powder sil MUATS**) | - | - | 69.75 | | - | - | - | - | - | - |
| 1.0 µm glass powder sil MUETS**) | - | - | - | 71.00 | - | - | - | - | - | - |
| 1.0 µm glass powder sil SI-1 | - | - | - | - | 76.00 | - | - | - | - | - |
| 1.0 µm glass powder sil SI-2 | - | - | - | - | - | 76.75 | - | - | - | - |

| | | | | | | | | | | |
|---|---|---|---|---|---|---|---|---|---|---|
| *) comparative example **) silane not according to formula (I) | | | | | | | | | | |

**Table 3: Flexural strength and modulus of cured composite C1-C10**

| **Composite** | **Particle size D₅₀ [µm]** | **Silane** | **Flexural strength (FS) and modulus (FM)¹⁾** | | | | **Flexural strength (FS) and modulus (FM)¹⁾** | | | |
|---|---|---|---|---|---|---|---|---|---|---|
| | | | **Stored dry at 23°C for 24 h** | | | | **Stored in water at 37°C for 24 h** | | | |
| | | | **FS [MPa]** | **SD²⁾** | **FM [GPa]** | **SD²⁾** | **FS [MPa]** | **SD²⁾** | **FM [GPa]** | **SD²⁾** |
| **C1*)** | 1.0 | MPTS**) | 155.7 | 9.1 | 10.59 | 0.65 | 161.0 | 12.6 | 10.46 | 0.44 |
| **C2** | 1.0 | MOTS | 179.4 | 13.2 | 11.73 | 0.65 | 173.6 | 12.1 | 12.02 | 0.37 |
| **C3*)** | 1.0 | MUATS**) | 158.9 | 12.4 | 9.78 | 0.54 | 160.6 | 10.6 | 10.30 | 0.74 |
| **C4*)** | 1.0 | MUETS**) | 164.4 | 8.3 | 10.49 | 0.54 | 159.5 | 13.3 | 10.18 | 0.64 |
| **C5** | 1.0 | SI-1 | 174.4 | 15.1 | 11.60 | 0.61 | 185.7 | 13.6 | 11.27 | 0.56 |
| **C6** | 1.0 | SI-2 | 175.3 | 13.7 | 11.48 | 0.81 | 172.2 | 16.3 | 11.56 | 0.35 |
| **C7** | 1.0 | MOTS | 159.4 | 8.4 | 10.83 | 0.51 | 168.2 | 9.7 | 11.37 | 0.60 |
| **C8*)** | 0.7 | MPTS**) | 118.1 | 12.6 | 8.17 | 0.35 | 145.5 | 9.7 | 9.36 | 0.37 |
| **C9** | 0.7 | MOTS | 131.0 | 11.1 | 9.95 | 0.36 | 152.7 | 7.7 | 10.15 | 0.62 |
| **C10*)** | 3.0 | MPTS**) | 137.7 | 12.0 | 12.54 | 0.87 | 153.8 | 10.4 | 12.78 | 0.59 |

| | | | | | | | | | | |
|---|---|---|---|---|---|---|---|---|---|---|
| *) comparative example **) silane not according to formula (I) ¹⁾ measured in accordance with ISO4049 dry at 23°C or after storage for 24 h in water at 37°C ²⁾ standard deviation | | | | | | | | | | |

### Example 10:

### Consistency measurements using a texture analyzer

Test samples were prepared by filling cylindrical steel molds (d = 8 mm, h = 4 mm) with composite. To obtain a flat surface, the excess of composite was removed. The resulting samples were heated up to desired temperature (23°C, 30°C, 60°C and 70°C) by placing the filled molds in a stainless-steel frame connected to thermostat (Julabo F12 ED Refrigerated/Heating Circulator, Julabo GmbH, Seelbach, Germany). The temperature of the samples was controlled using an IR thermometer (Fluke 62 MAX+). The compressive force of the samples was determined using a texture analyzer (TA.XTplusC, Stable Micro Systems, Godalming, United Kingdom) in combination with a flat-ended cylindrical plastic probe (d = 6 mm) connected to the force transducer of the analyzer. The flat-ended plastic probe was mechanically lowered with a velocity of 0.1 mm/s into the sample. As soon as a force of 6.8 g was detected, the data-acquisition was started, and the data were recorded until a penetration depth of 2 mm was reached. The compressive force was defined as the maximal measured force of the obtained force vs penetration depth plot. The compressive force was measured 3 times for each composite. The state-of-the-art packable composites Tetric EvoCeram, Tetric PowerFill and Tetric Prime were tested as reference materials. The test results are shown in Table 4.

At room temperature (23°C) and at 30°C, composites C1-C10 had a consistency (absolute positive force) of ≥ 500 g, which is similar to that of the commercially available composites Tetric EvoCeram, Tetric PowerFill and Tetric Prime (Ivoclar Vivadent AG, Schaan, Liechtenstein) and which is typical for packable materials. At 60°C, the composites C2, C5, C6, C7, C9 and comparative material C10 had a consistency (absolute positive force) of ≤ 60 g, which is typical for a flowable composite, while the comparative materials C1, C3, C4 and C8 still had a consistency comparable to that of the commercially available packable composites (absolute positive force > 60 g).

The comparative materials C1, C3 and C4 differ from composites C2, C5 and C6 in that no silane according to formula I was used for the surface modification of the filler. Although the composites C2, C5 and C6 have a higher filler content, their consistency at 60°C is surprisingly lower that that of the comparative materials.

Similar results were found for composite C9 and the comparative material C8. Surface modification of the filler with a silane according to formula (I) significantly reduced the consistency at 60°C although a higher filler content was used.

**Table 4: Consistency of composites C1-C7 and commercial products**

| **Composite** | **Particle Size D₅₀ [µm]** | **Silane** | **Absolute positive force (FS) [g]** | | | | | |
|---|---|---|---|---|---|---|---|---|
| | | | **23°C** | **SD¹⁾** | **30°C** | **SD¹⁾** | **60°C** | **SD¹⁾** |
| Tetric EvoCeram*) | - | - | 3334.6 | 84.6 | 1528.5 | 53.0 | 146.8 | 14.3 |
| Tetric PowerFill*) | - | - | 3196.4 | 259.0 | 1423.6 | 96.1 | 77.9 | 4.9 |
| Tetric Prime*) | - | - | 1878.5 | 188.0 | 664.9 | 42.9 | 81.7 | 4.7 |
| C1*) | 1.0 | MPTS**) | 3153.0 | 106.9 | 1848.6 | 75.2 | 210.9 | 11.8 |
| C2 | 1.0 | MOTS | 2958.4 | 81.6 | 899.4 | 42.5 | 38.5 | 3.3 |
| C3*) | 1.0 | MUATS**) | 3183.5 | 69.2 | 1664.5 | 58.6 | 171.8 | 13.7 |
| C4*) | 1.0 | MUETS**) | 3401.5 | 79.9 | 1539.8 | 93.6 | 84.9 | 6.7 |
| C5 | 1.0 | SI-1 | 2854.6 | 31.6 | 722.8 | 2.1 | 9.7 | 1.1 |
| C6 | 1.0 | SI-2 | 2720.3 | 77.4 | 677.4 | 29.4 | 17.7 | 0.6 |
| C7 | 1.0 | MOTS | 2778.7 | 134.6 | 597.1 | 5.9 | 11.2 | 0.6 |
| C8*) | 0.7 | MPTS**) | 3058.1 | 104.9 | 966.6 | 14.9 | 80.5 | 6.9 |
| C9 | 0.7 | MOTS | 2605.6 | 41.8 | 574.6 | 38.4 | 21.3 | 2.0 |
| C10*) | 3.0 | MPTS**) | 2758.8 | 123.2 | 1206.1 | 11.3 | 51.7 | 6.1 |

| | | | | | | | | |
|---|---|---|---|---|---|---|---|---|
| *) comparative example **) silane not according to formula (I) ¹⁾ standard deviation | | | | | | | | |

A comparison of composite C9 with the comparative material C10 further shows, that increasing the particle size from 0.7 to 3.0 µm also results in a reduction of the consistency at 60°C. However, larger particles are disadvantageous because they increase surface roughness and impair the polishability of the hardened materials.

The composite C7 differs from composite C2 in that the monomer mixture additionally comprises a chain transfer agent, which caused a further reduction of the consistency at 60°C and thus further improves the flow properties.

At 60°C, the consistency of composite C3 is significantly higher than that of composite C4. The silane used to modify the filler of composite C3 comprises a urea group. This comparison shows that urea groups are less effective in reducing consistency at elevated temperatures. Urethane groups, on the other hand, can be used to adjust the temperature effect on the consistency as is evident from a comparison of composites C5 and C6 with composite C2.

A comparison of composite C2 with the comparative material C1, as well as a comparison of composites C5 and C6 with the comparative material C4 shows that the spacer length between the silane group and the methacrylate group of the silanes is an essential parameter. Short spacers do not give the desired effect, whereas longer spacers provide the desired consistency change upon heating. Although the spacer of C4 comprises a urethane group, the performance of this composite is still worse than that of composites C5 and C6 for this reason.

### Example 11:

### Determination of the shrinkage force of composites C2 and C7

Shrinkage force measurements were performed by using a Bioman instrument in combination with a flat ended steel rod (height: 25 mm, diameter: 10 mm) connected to the cantilever load cell of the Bioman instrument. On the stationary part of the machine framework, a glass slide (3 × 25 × 75 mm) was fixed. The flat end of the steel rod and the midsection of the glass slide were treated with a primer (steel rod: SR Bond; glass slide: Monobond S, Ivoclar Vivadent AG). The primer provides a strong adhesion between the treated surfaces and the composite. The composite to be tested (0.2 g) was deposited on the glass slide. The layer thickness of the composite between the steel rod and the glass slide was set to 0.8 mm. The excess of the composite was removed, and the remaining composite was irradiated for 10 s using an LED curing light (Bluephase PowerCure; Ivoclar Vivadent AG), which was installed below the glass slide. The shrinkage force was monitored as a function time over 10 min. Each sample was measured twice. The test results are shown in Table 5.

The composite C7, containing the chain transfer agent (CTE) ASEE, showed a significantly lower shrinkage force compared to composite C2 without CTA.

**Table 5: Shrinkage force of composites C2 and C7**

| **Composite** | **Silane** | **Shrinkage force [N]** | |
|---|---|---|---|
| | | **1800 s** | **SD¹⁾** |
| **C2** | MOTS | 105.0 | 0.5 |
| **C7** | MOTS | 92.6 | 2.1 |

| | | | |
|---|---|---|---|
| ¹⁾ standard deviation | | | |

### Example 12

### Evaluation of the gloss stability of the prepared dental composites

To determine the polishability of the dental materials, the material to be tested was filled into a cylindrical metal mold (d=10 mm, h=5-10 mm) (η=8 per series). The material was then exposed 2 times for 20 s to light in the wavelength range of 380 to 520 nm (light power 1200 mW/cm²). The cured specimens were then bonded to an SEM support and end-polymerized using a LED cube 100 (channel 1 (λ = 460 nm) + channel 2 (λ = 410 nm) at 60 % intensity, Hönle) controlled by a LED Powerdrive (Hönle) for 200 s. After the specimens were stored dry for 24 h at 37° C, each test specimen was polished with a polisher (OptraGloss TIP single-step polisher) after standardized roughening (320 grit abrasive paper, Buehler) with a defined contact pressure of 2 N. The polishing was performed under water supply with a dental handpiece (KaVo) and a rotational speed of 10 000 rpm. Each specimen was polished for 10 s/20 s/30 s. After each polishing interval, the specimen was rinsed with water spray, dried with the air blower, and both surface gloss and surface roughness were measured. The surface gloss was determined with a glossmeter (Novo-curve, Rhopoint, Bexhill-on-Sea, UK). The measuring principle of the glossmeter is that a light beam hits the surface to be measured at an angle of 60° and the intensity of the reflected light is measured in the instrument and compared with a reference value. The higher the value, the higher the gloss. Gloss was measured in gloss units (GU) relative to a calibration glass (black glass plate with a value of 93.7 GU). Black foam has a value of 0 GU. The test results are shown in Table 6.

To measure the surface roughness Rₐ (mean surface roughness Rₐ in µm), the samples were illuminated with focused white light using a special optical sensor (CWL). An internal optical device, whose focal length has a strong wavelength dependence, splits the white light into different colors (corresponding to the different wavelengths). A miniaturized spectrometer detects the color of the light reflected from the sample and uses an internal calibration table to determine the vertical position on the sample surface. The xy position is measured with rotary encoders. The measurement was performed with a resolution of 10 nm in height and 1 to 2 pm lateral resolution at a maximum measurement frequency of 1000 E1ζ. The average roughness Rₐ (in µm) is the arithmetic mean of the profile ordinates. The measurement is preferably performed with the FRT MicroProf measuring instrument (FRT MicroProf, Fries Research & Technology GmbFl, Bergisch Gladbach, De). The results are shown in Table 7.

**Table 6: Polishability of composites C8, C9 and C10**

| **Composit es** | **Glass filler D₅₀ [µm]** | **Polishability [GU]** | | | | | | | |
|---|---|---|---|---|---|---|---|---|---|
| | | **0 s** | **SD¹⁾** | **10s** | **SD¹⁾** | **20 s** | **SD¹⁾** | **30 s** | **SD¹⁾** |
| **C8*)** | 0.7 | 3.4 | 0.7 | 78.2 | 7.5 | 81.5 | 5.6 | 84.0 | 1.9 |
| **C9** | 0.7 | 4.2 | 0.5 | 78.0 | 3.5 | 82.5 | 1.8 | 84.3 | 1.7 |
| **C10*)** | 3.0 | 2.1 | 0.2 | 22.5 | 3.8 | 37.1 | 1.0 | 48.9 | 3.1 |

| | | | | | | | | | |
|---|---|---|---|---|---|---|---|---|---|
| *) comparative example ¹⁾ standard deviation | | | | | | | | | |

**Table 7: Surface roughness of composites C8, C9 and C10**

| **Composites** | **Glass filler D₅₀ [µm]** | **Surface roughness Rₐ [µm]** | | | | | | | |
|---|---|---|---|---|---|---|---|---|---|
| | | **0 s** | **SD¹⁾** | **10s** | **SD¹⁾** | **20 s** | **SD¹⁾** | **30 s** | **SD¹⁾** |
| **C8*)** | 0.7 | 0.87 | 0.06 | 0.14 | 0.03 | 0.12 | 0.02 | 0.12 | 0.02 |
| **C9** | 0.7 | 0.93 | 0.09 | 0.14 | 0.03 | 0.12 | 0.3 | 0.14 | 0.04 |
| **C10*)** | 3.0 | 0.98 | 0.07 | 0.37 | 0.06 | 0.29 | 0.04 | 0.27 | 0.03 |

| | | | | | | | | | |
|---|---|---|---|---|---|---|---|---|---|
| *) comparative example ¹⁾ standard deviation | | | | | | | | | |

A comparison between C9 and C10 shows that both the polishability and the surface roughness of the dental composite C9 comprising filler with a particle size of 0.7 µm were better than for composite C10, containing larger particles (3.0 µm). The polishability and the surface roughness are important parameters for the esthetic properties of dental composites. A comparison of composite C9 with the reference composite C8 shows that both composites exhibit comparable polishability and surface roughness. The silane according to the invention therefore does not impair these properties.

## Claims

1. A radically polymerizable composition comprising
(a) at least one urethane di(meth)acrylate monomer;
(b) at least one aromatic di(meth)acrylate monomer;
(c) at least one inorganic filler; and
(d) at least one photoinitiator system for the radical polymerization,
**characterized in that** the inorganic filler (c) has an average particle size within the range of from 0.15 to 1.5 µm and is surface-modified with a silane according to formula (I), 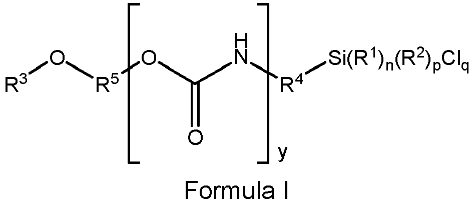 wherein
R¹ is a OCH₃ or a OC₂H₅ group;
R² is a CH₃ or a C₂H₅ group;
R³ is a group according to formula (II), wherein R⁶ is H or preferably CH₃, or a group according to formula (III), wherein R⁷ is a C₁-C₈ alkyl group, preferably a C₁-C₆ alkyl group, and more preferably a C₁-C₄ alkyl group; 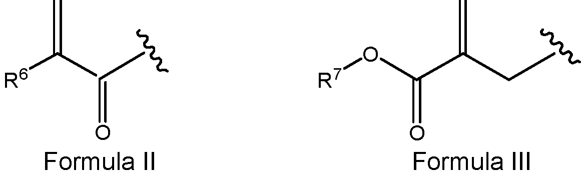
R⁴ is a linear or branched C₂-C₁₀ alkanediyl group, preferably a C₂-C₆ alkanediyl group, and more preferably a C₂-C₄ alkanediyl group;
R⁵ is a linear or branched C₆-C₁₄ alkanediyl group, preferably a C₆-C₁₂ alkanediyl group, and more preferably a C₆-C₁₀ alkanediyl group;
n = 0, 1, 2 or 3;
p = 0, 1 or 2;
q = 0, 1, 2 or 3;
y = 0 or 1; and
n + p + q = 3.

2. The radically polymerizable composition according to claim 1, wherein the at least one urethane di(meth)acrylate monomer (a) is a monomer according to formula (IV): 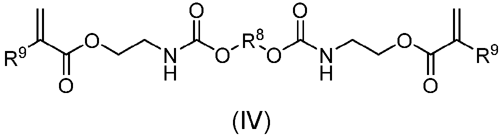 wherein
R⁸ is a branched or linear C₂-C₁₂ alkanediyl group, preferably a C₂-C₁₀ alkanediyl group, and more preferably a C₂-C₈ alkanediyl group, that can be interrupted by one or more O atoms and/or -C(O)O- groups, and
R⁸ is H or preferably CH₃;
and/or formula (V): 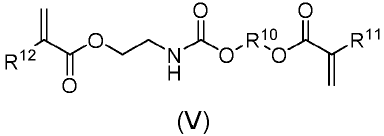 wherein
R¹⁰ is a branched or linear C₂-C₁₀ alkanediyl group, preferably a C₂-C₈ alkanediyl group, and more preferably a C₂-C₆ alkanediyl group, that can be interrupted by one or more O atoms and/or -C(O)O- groups,
R¹¹ is H or preferably CH₃, and
R¹² is H or preferably CH₃;
and/or formula (VI): 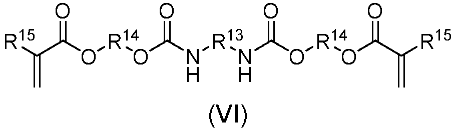 wherein
R¹³ is a branched or a linear C₂-C₁₆ alkanediyl group or a C₆-C₂₀ cycloaliphatic group, preferably a C₄-C₁₂ alkanediyl group or a C₆-C₁₆ cycloaliphatic group, and more preferably a C₅-C₁₀ alkanediyl group or a C₆-C₁₃ cycloaliphatic group,
R¹⁴ is a branched or linear C₂-C₁₀ alkanediyl group, preferably a C₂-C₈ alkanediyl group, and more preferably a C₂-C₆ alkanediyl group, that can be interrupted by one or more O atoms and/or -C(O)O- groups, and
R¹⁵ is H or preferably CH₃.

3. The radically polymerizable composition according to claim 1 or 2, wherein the at least one aromatic di(meth)acrylate monomer (b) is
an urethane di(meth)acrylate derivative according to the following formula V380: 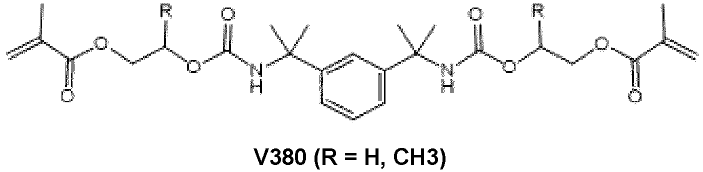
wherein the radicals R are independently H or CH₃, and may have the same meaning or different meanings, or mixture thereof; and/or
a monomer according to formula (VII): 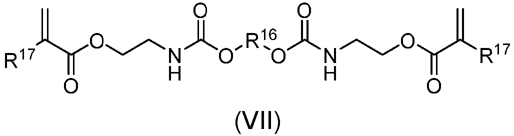
wherein
R¹⁶ is a C₇-C₃₀-alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R¹⁸, where R¹⁸ is a branched or preferably linear C₁-C₂₀ alkyl group and/or acyl groups -C(O)-R¹⁹, where R¹⁹ is a branched or preferably linear C₁-C₂₀ alkyl group; and
R¹⁷ is H or preferably CH₃; and/or
a monomer according to formula (VIII): 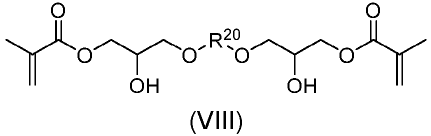
wherein
R²⁰ is a C₆-C₃₀-aryl or alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R²¹, where R²¹ is a branched or preferably linear C₁-C₂₀ alkyl group, and/or acyl groups -C(O)-R²², where R²² is a branched or preferably linear C₁-C₂₀ alkyl group.

4. The radically polymerizable composition according to any one of claims 1 to 3, wherein the inorganic filler (c) is a radiopaque glass filler, preferably a barium or strontium aluminum silicate glasses and/or a fluoroaluminosilicate (FAS) glass filler,
preferably a radiopaque glass fillers having the following composition (wt.%): SiO₂: 20-80; B₂O₃: 2-15, BaO or SrO: 10-40; Al₂O₃: 2-20; CaO and/or MgO: 0-20; Na₂O, K₂O, Cs₂O: 0-10 each; WOs: 0-20; ZnO: 0-20; La₂O₃: 0-10; ZrO₂: 0-15; P₂O₅: 0-30; Ta₂O₅, Nb₂O₅ or Yb₂O₃: 0-5; and CaF₂ and/or SrF₂ 0-10. Particularly preferred are radiopaque glass fillers having the composition (wt.%): SiO₂: 40-75; B₂O₃: 2-15; BaO or SrO: 15-35; Al₂O₃: 2-15; CaO and/or MgO: 0-10; and Na₂O: 0-10; and/or
a fluoroaluminosilicate (FAS) glass fillers having the following composition (wt.%): SiO₂: 20-35; Al₂O₃: 15-35; BaO or SrO: 10-25; CaO: 0-20; ZnO: 0-15; P₂O₅: 5-20; Na₂O, K₂O, Cs₂O: 0-10 each; and CaF₂: 0.5-20. Particularly preferred are FAS fillers with the composition (wt.%): SiO₂: 20-30; Al₂O₃: 20-30; BaO or SrO: 10-25; CaO: 5-20; P₂O₅: 5-20; Na₂O: 0-10; and CaF₂: 5-20,
whereby all weight percentages refer to the total weight of the glasses and, with the exception of fluorides, are calculated on the basis of the oxides.

5. The radically polymerizable composition according to any one of claims 1 to 4, further comprising one or more additional dimethacrylate monomers (e) different from the monomers (a) and (b) and/or one or more monofunctional (meth)acrylate monomers (f),
preferably at least one dimethacrylate monomer (e) which is selected from the group consisting of ethoxylated or propoxylated bisphenol A dimethacrylate, 2,2-bis[4-(2-methacryloyloxy-propoxy)phenyl]propane, di-, tri- or tetraethylene glycol dimethacrylate, glycerol dimethacrylate, 1,4-butanediol dimethacrylate, 1,10-decanediol dimethacrylate (DsMA), polyethylene glycol or polypropylene glycol dimethacrylates, or 1,12-dodecanediol dimethacrylate.

6. The radically polymerizable composition according to any one of claims 1 to 5 further comprising at least one chain transfer agent (CTA) (g), preferably an allyl sulfone and/or allyl sulfide according to formula (IX): 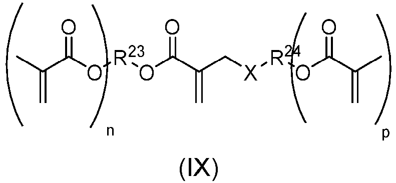 wherein
X is SO₂ and
R²³ is a branched or preferably linear C₁-C₁₆, preferably C₁-C₁₄ and more preferably C₁-C₁₂ alkyl group that can be interrupted by one to three, preferably one to two O atoms,
R²⁴ is a tolyl group (in this case, p = 0) or a branched or preferably linear C₁-C₁₆, preferably C₁-C₁₄ and more preferably C₁-C₁₀alkyl group that can be interrupted by one to three, preferably one to two O atoms and that can also be interrupted by either a -(C=O)-O- ester group or a -O(C=O)-NH-urethane group and that can be functionalized by one or two OH groups,
n is 0 or 1, and
p is 0 or 1;
or
X is S and
R²³ is a branched or preferably linear C₁-C₁₀, preferably C₁-C₆ and more preferably C₁-C₄ alkyl group,
R²⁴ is a branched or preferably linear C₁-C₁₆, preferably C₁-C₁₄ and more preferably C₁-C₁₀ alkyl group that can be interrupted by one to three, preferably one to two O atoms and that can also be interrupted by either a -(C=O)-O- ester group or a -O(C=O)-NH- urethane group and that can be functionalized by one or two OH groups,
n is 0 or 1, and
p is 0 or 1.

7. The radically polymerizable composition according to any one of claims 1 to 6, comprising
(a) 2 to 30 wt.%, more preferably, 3 to 25 wt.%, and most preferably 4 to 20 wt.% of the at least one urethane di(meth)acrylate monomer;
(b) 3 to 30 wt.%, more preferably, 4 to 25 wt.%, and most preferably 5 to 20 wt.% of the at least one aromatic di(meth)acrylate monomer;
(c) 30 to 90 wt.% , more preferably, 35 to 85 wt.%, and most preferably 40 to 80 wt.% of the at least one glass filler that is at least partially functionalized with a silane according to the formula (I); and
(d) 0.05 to 2.0 wt.%, more preferably, 0.08 to 1.0 wt.%, and most preferably 0.1 to 0.8 wt.% of the at least one photoinitiator system for the radical polymerization,
whereby all percentages refer to the total weight of the composition.

8. The radically polymerizable composition according to claim 7,
wherein the at least one **urethane di(meth)acrylate monomer (a)** is a compound according to formula (IV) or (V) and is preferably selected from the group consisting of bis-[(2-methacryloyloxyethoxy-carbonyl)-amino]-2,2,4-trimethylhexane (UDMA) and V837, which is the reaction product of 2-isocyanatoethyl methacrylate with 2-hydroxyethyl methacrylate, and mixtures thereof;
the at least one **aromatic di(meth)acrylate monomer (b)** is V380 or a compound according to formula (VIII) and is preferably selected from the group consisting of 2-methyl-acrylic acid 2-[1-methyl-1-(3-{1-methyl-1-[2-(2-methyl-acryloyloxy)-ethoxycarbonylamino]-ethyl}-phenyl)-ethylcarbamoyloxy]propyl ester, 2,2-bis[4-(2-hydroxy-3-methacryloyloxypropoxy)-phenyl]propane (Bis-GMA), and mixtures thereof;
the at least one **glass filler (c)** is a barium or strontium aluminum silicate glass filler and/or fluoroaluminosilicate (FAS) glass filler that is at least partially functionalized with a silane according to the formula (I), wherein
R¹ is a OCH₃ group;
R² is a CH₃ or a C₂H₅ group;
R³ is a group according to formula (II), wherein R⁶ is CH₃;
R⁴ is a linear C₂-C₄ alkanediyl group;
R⁵ is a linear C₆-C₁₀ alkanediyl group;
n = 3;
p = 0;
q = 0;
y = 0 or 1.
and
the at least one photoinitiator system (d) for the radical polymerization comprises a Norrish type 1 or Norrish type 2 photoinitiator

9. The radically polymerizable composition according to claim 7 or 8, additionally comprising
(e) 0 to 25 wt.%, more preferably, 0 to 20 wt.%, and most preferably 2 to 20 wt.% of at least one dimethacrylate monomer that is different to monomers (a) and (b); and/or
(f) 0 to 25 wt.% , more preferably, 0 to 20 wt.%, and most preferably 0 to 15 wt.% of at least one monofunctional (meth)acrylate monomer; and/or
(g) 0.3 to 10 wt.% , more preferably, 0.4 to 7 wt.%, and most preferably 0.5 to 5 wt.% of at least one chain transfer agent, preferably an allyl sulfide or an allyl sulfone; and/or
(h) 0 to 30 wt.% , more preferably, 0 to 25 wt.%, and most preferably 3 to 20 wt.% of at least one mixed oxide filler; and/or
(i) 0 to 30 wt.% , more preferably, 3 to 25 wt.%, and most preferably 5 to 20 wt.% of one or more additional fillers,
whereby all percentages refer to the total weight of the composition.

10. The composition of any one of claims 1 to 9 for use in a method for the treatment of damaged teeth.

11. The composition according to claim 10 for use in the treatment of teeth damaged by caries.

12. Use of a composition according to any one of claims 1 to 9 for use in the extra-oral restoration of dentures or artificial teeth.
